# EUROPEAN PATENT APPLICATION

(11) **EP 4 512 899 A1**
(43) Date of publication of application: **26.02.2025**
(21) Application number: 23193046.2
(22) Date of filing: 23.08.2023
(51) Int. Cl.: C12N 15/113, A61K 31/7115, A61K 31/7125, A61K 31/712, A61P 3/00, A61P 9/00

(54) **ANGPTL4 ASO COMPOSITIONS FOR TREATMENT OF ATHEROSCLEROSIS IN HUMANS**

(71) Applicant: Lipigon Pharmaceuticals AB, 90736 Umeå (SE)
(72) Inventor: NILSSON, Stefan K., 907 52 Umeå (SE); OLIVECRONA, Gunilla, 903 21 Umeå (SE); KOOIJMAN, Sander, 2717 DD Zoetermeer (NL); RENSEN, Patrick C.N., 2316 AB Leiden (NL); SCHÖNKE, Milena, 2333 BG Leiden (NL)
(74) Representative: Zacco Denmark A/S

(57) **Abstract**

The disclosure relates to ANGPTL4 inhibitors and to the use of such inhibitors in treatment, of a cardiovascular diseases.

## Description

### Technical field

The present disclosure relates to an angptl4 inhibitor, and a pharmaceutical composition comprising an angptl4 inhibitor. More specifically, the disclosure relates to an angptl4 inhibitor, and a pharmaceutical composition comprising such an inhibitor for use in treating cardiovascular diseases, such as for regulation of factors important for development of cardiovascular disease. In some instances, the ANGPTL4 inhibitor is for use in combination with an ANGPTL3 inhibitor.

### Background art

Angiopoietin-like 3 (ANGPTL3) and 4 (ANGPTL4) inhibit lipoprotein lipase (LPL) to regulate tissue fatty acid uptake from triglyceride (TG)-rich lipoproteins (TRLs). While pharmacological inhibition of ANGPTL3 is under clinical investigation as a lipid-lowering strategy, systemic ANGPTL4 inhibition has not previously been pursued, due to expected adverse effects. ANGPTL4 is expressed in a variety of tissues and inhibits LPL activity predominantly during fasting when its gene expression is elevated. Thereby, LPL activity in white adipose tissue (WAT) is low during fasting, preventing TRL-derived TG storage [1, 2]. In brown adipose tissue (BAT), ANGPTL4 dictates diurnal and cold-induced LPL activity to replenish intracellular lipid stores following sustained thermogenesis [3, 4].

Since the discovery that human loss-of-function gene variants of *ANGPTL3* and *ANGPTL4* associate with lowered plasma TG levels and reduced CVD risk [5, 6], therapeutic silencing of these LPL inhibitors received much attention. Monoclonal antibodies (mAbs) and antisense oligonucleotides (ASOs) targeting ANGPTL3 have been successfully shown to reduce plasma TG and LDL-C in mice and humans [7-11]. In contrast, global inhibition of ANGPTL4 is not considered a viable therapeutic strategy due to adverse effects such as the formation of lipogranulomatous lesions in the intestines and chylous ascites, likely consequential from the heterogeneous expression and function of ANGPTL4 across tissues.

The present invention provides solutions to these problems with global inhibition of ANGPTL4, while still providing significant pharmacological effects on various plasma lipids.

### Summary

It is an object of the present disclosure to mitigate, alleviate or eliminate one or more of the above-identified deficiencies and disadvantages in the prior art and solve at least the above mentioned problem of lowering a number of disease causing plasma lipids.

This is achieved by providing potent anti ANGPTL4 antisense oligonucleotide compounds that are targeted to the liver. The present inventors has found that targeting the anti ANGPTL4 compounds to the liver of mammals, such as i.e. primates, does not cause any of the expected side effects. Further, such liver targeted ANGPTL4 inhibitors still cause significant and durable down regulation of various plasma lipids involved in development of cardiovascular disease, and indeed are able to reduce i.e. development of atherosclerosis.

According to a first aspect there is provided an ANGPTL4 inhibitor comprising an antisense oligonucleotide consisting of 10-50 nucleotides in length, wherein 10-30 consecutive nucleotides are complementary to SEQ ID NO.1 or SEQ ID NO. 2, and has at least one affinity-enhancing nucleotide analogue and comprises at least one phosphorothioate internucleoside linkage, and wherein the ANGPTL4 inhibitor is for use in treatment, alleviation, pre-emptive treatment, prevention or prophylaxis of a cardiovascular disease in a mammal.

The inventors of the present invention has found that *in vivo* administration of inhibitors of ANGPTL4 or even ANGPTL3 and 4 inhibitors combined are able to influence certain factors important for development of cardiovascular disease.

The parameters listed in the above embodiments, and which can all be reduced by administration of the ANGPTL4 compounds of the invention are disease parameters which can be measured in circulation of a patient and which will be monitored by a medical doctor in a clinical setting in order to evaluate risk of e.g. atherosclerosis. Indeed, the inventors has shown that the compounds of the invention are capable of significantly influencing the development of atherosclerosis in a mammal.

According to some embodiments, the use of the ANGPTL4 compounds of the invention, such as the compositions comprising those compounds, is for prevention of atherosclerotic lesions.

According to some embodiments, the ANGPTL4 inhibitor is for use in combination with an ANGPTL3 inhibitor. The inventors have found that combining ANGPTL4 inhibitors of the invention with an ANGPTL3 inhibitor provide advantageously good results in downregulation of Total Cholesterol, non-HDL-C or plasma Triglyceride levels in circulation even when homing the ANGPTL3 and 4 inhibitors to the liver. Such combination treatment also provide reductions in these factors when administered long term. According to some embodiments, the ANGPTL3 inhibitor is an ANGPTL3 antibody and/or an ANGPTL3 antisense oligonucleotide

According to a second aspect there is provided an ANGPTL4 inhibitor comprising the antisense oligonucleotide with the sequence
+A*+G*+T*A*C*T*G*G*C*C*G*T*T*G*+A*+G*+G, (SEQ ID NO. 186) wherein + are LNA such as beta-deoxy LNA, * are phosphorothioate internucleotide linkages, C is methyl-Cytocine, and wherein the inhibitor is attached to a homing moiety targeted to the liver.

Expectedly, systemic administration to mammals such as humans, of an ANGPTL4 inhibitor would cause side effects, however, the present inventors found that these expected side effects, were abolished by addition of a liver homing moiety to the ANGPTL4 antisense oligonucleotide, while still achieving a pharmacological effect on important disease parameters in circulation. Further, this improved compound will provide a pharmacological effect when administered in an effective dosage for at least 4 weeks.

According to some embodiments of the invention, the homing moiety is a GalNac molecule, such as a tri-antennary GalNac molecule. According to some embodiments, the homing moiety attached to the inhibitor (the ANGPTL4 antisense oligonucleotide) by a linker.

According to some embodiments, the linker is any one of a nucleotide linker or an amino linker. According to some embodiments, the linker is a nucleotide linker with a length of between 1 and 20 nucleotides. According to some embodiments, the linker is an amino linker with the formula NH-(CH2)2-12. According to some embodiments, the linker is a NH-(CH2)6, or NH-(CH2)8 or NH-(CH2)10 linker.

According to some embodiments, the antisense oligonucleotide consist of the sequence +A*+G*+T*A*C*T*G*G*C*C*G*T*T*G*+A*+G*+G, wherein + are LNA such as beta-deoxy LNA, * are phosphorothioate internucleotide linkages, C is methyl-Cytocine, and wherein the inhibitor is attached to a liver targeting homing moiety wherein linker and homing moiety has the structure

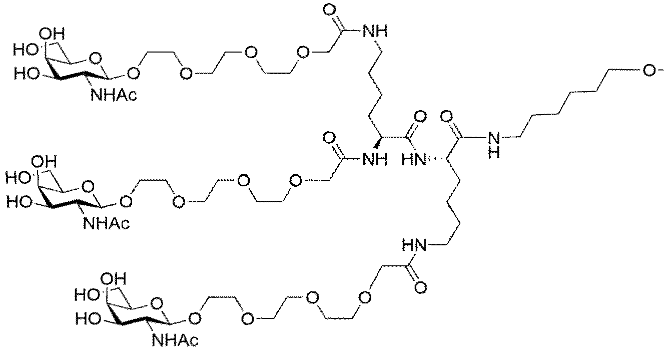

The inventors has shown that this liver targeted ANGPTL4 inhibitor has surprising pharmacological effects by being able to significantly reduce important plasma lipid levels and furthermore lack the expected toxic effects.

According to a third aspect there is provided a pharmaceutical compositions comprising the ANGPTL4 inhibitor of the invention. According to some embodiments, the invention provides for a method of treatment using the ANGPTL4 inhibitor according to the invention. According to some embodiments, the ANGPTL4 inhibitor according to the invention and the previous embodiments is for use in treatment, alleviation, pre-emptive treatment, prevention or prophylaxis of a cardiovascular disease in a mammal. According to some embodiments, the mammal is a human.

The inventors have surprisingly shown, that the ANGPTL4 inhibitors according to the invention can be used safely, for treatment of cardiovascular diseases in a mammal such as in a human. According to some embodiments, the cardiovascular disease is atherosclerosis. According to some embodiments, the use is for lowering of plasma triglycerides and/or total cholesterol, such as for treatment of high levels of plasma triglycerides and/or high levels of total cholesterol. According to some embodiments, the use is for reducing circulating triglyceride-rich lipoproteins, such as for treatment of high levels of circulating triglyceride-rich lipoproteins. According to some embodiments, the use is for increasing plasma HDL-C levels, such as for treatment of low plasma HDL-C levels. According to some embodiments, the use is for reducing VLDL-ApoB production, such as for treatment of high VLDL-ApoB levels.

According to some embodiments, the use is for prevention of atherosclerotic lesions, such as for reducing atherosclerotic lesion size, such as for reducing the number of severe atherosclerotic lesions, such as for improving the atherosclerotic lesion index in a patient.

According to some embodiments, the ANGPTL4 inhibitor of the second aspect is for use in combination with another drug. In some embodiments, such other drug may be another drug used for treatment of hyperlipidemia, or for prevention of cardiovascular disease. According to some embodiments, the inhibitor is for use in combination with an ANGPTL3 inhibitor, which in some embodiments may be an antisense oligonucleotide or an antibody against ANGPTL3.

The present disclosure will become apparent from the detailed description given below. The detailed description and specific examples disclose preferred embodiments of the disclosure by way of illustration only. Those skilled in the art understand from guidance in the detailed description that changes and modifications may be made within the scope of the disclosure.

It is to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context explicitly dictates otherwise. Thus, for example, reference to "a unit" or "the unit" may include several devices, and the like. Furthermore, the words "comprising", "including", "containing" and similar wordings does not exclude other elements or steps.

### Terminology

The term "GalNac" is to be interpreted as *N*-acetylgalactosamine.

The term "ASO" or "antisense oligonucleotide" is an oligonucleotide according to the invention which is complementary to its target RNA, in the present invention, it will be complementary to ANGPTL4 or ANGPTL3 RNA or mRNA.

In describing the embodiments of the invention, specific terminology will be resorted to for the sake of clarity. However, the invention is not intended to be limited to the specific terms so selected, and it is understood that each specific term includes all technical equivalents, which operate in a similar manner to accomplish a similar purpose.

The term "therapeutically effective amount", or "effective amount" or "effective dose", refers to an amount of a therapeutic agent, which confers a desired therapeutic effect on an individual in need of the agent. The effective amount may vary among individuals depending on the health and physical condition of the individual to be treated, the taxonomic group of the individuals to be treated, the formulation of the composition, the method of administration, assessment of the individual's medical condition, and other relevant factors.

The term "treatment" refers to any administration of a therapeutic medicament, herein comprising an antisense oligonucleotide that partially or completely cures or reduces one or more symptoms or features of a given disease.

The term "compound" as used herein, refers to a compound comprising an oligonucleotide according to the invention. In some embodiments, a compound may comprise other elements apart from the oligonucleotide of the invention. Such other elements may in non-limiting example be a delivery vehicle which is conjugated or in other way bound to the oligonucleotide.

"Antisense oligonucleotide" means a single-stranded oligonucleotide having a nucleobase sequence that permits hybridization to a corresponding region or segment of a target nucleic acid. The antisense oligonucleotide of the present invention is preferably a "mixmer".

The term "antisense oligonucleotides complementary to miR-134" is used interchangeably with the terms "antimiR-134" or "antimiR-134 oligonucleotide" or "antimiR-134 antisense oligonucleotide".

A "gapmer" is an antisense oligonucleotide, comprising a mix of nucleoside analogues such as LNA and DNA nucleosides, and wherein the antisense oligonucleotide comprises an internal region having a plurality of nucleosides (such as a region of at least 5 or 6 or 7 DNA nucleotides), capable of recruiting an RNAse, such as RNAseH, wherein the nucleosides comprising the internal region are chemically distinct from the nucleoside or nucleosides comprising the external wings.

"Nucleoside analogues" are described by e.g. Freier & Altmann; Nucl. Acid. Res., 1997, 25, 4429 - 4443 and Uhlmann; Curr. Opinion in Drug Development, 2000, 3(2), 293-213, and examples of suitable and preferred nucleoside analogues are provided by WO2007031091, which are hereby incorporated by reference.

"5-methylcytosine" means a cytosine modified with a methyl group attached to the 5' position. A 5-methylcytosine is a modified nucleobase.

"2'-O-methoxyethyl" (also 2'-MOE and 2'-O(CH~)~-OCH3) refers to an O-methoxy-ethyl modification at the 2' position of a furanose ring.

"2'-MOE nucleoside" (also 2'-O-methoxyethyl nucleoside) means a nucleoside comprising a 2'-MOE modified sugar moiety.

A "locked nucleic acid" or "LNA" is often referred to as inaccessible RNA, and is a modified RNA nucleobase. The ribose moiety of an LNA nucleobase is modified with an extra bridge connecting the 2' oxygen and 4' carbon. An LNA oligonucleotide offers substantially increased affinity for its complementary strand, compared to traditional DNA or RNA oligonucleotides. In some aspects bicyclic nucleoside analogues are LNA nucleotides, and these terms may therefore be used interchangeably, and in such embodiments, both are characterized by the presence of a linker group (such as a bridge) between C2' and C4' of the ribose sugar ring. When used in the present context, the terms "LNA unit", "LNA monomer", "LNA residue", "locked nucleic acid unit", "locked nucleic acid monomer" or "locked nucleic acid residue", refer to a bicyclic nucleoside analogue. LNA units are described in inter alia WO 99/14226, WO 00/56746 , WO 00/56748 , WO 01/25248 , WO 02/28875 , WO 03/006475, WO2015071388, and WO 03/095467.

"Beta-D-Oxy LNA", is a preferred LNA variant.

"Bicyclic nucleic acid" or "BNA" or "BNA nucleosides" mean nucleic acid monomers having a bridge connecting two carbon atoms between the 4' and 2' position of the nucleoside sugar unit, thereby forming a bicyclic sugar. Examples of such bicyclic sugar include, but are not limited to A) pt-L-methyleneoxy (4'-CH2-0-2') LNA, (B) P-D-Methyleneoxy (4'-CH2-0-2') LNA, (C) Ethyleneoxy (4'- (CH2)2-0-2') LNA, (D) Aminooxy (4'-CH2-0-N(R)-2') LNA and (E) Oxyamino (4'-CH2-N(R)-0-2') LNA.

As used herein, LNA compounds include, but are not limited to, compounds having at least one bridge between the 4' and the 2' position of the sugar wherein each of the bridges independently comprises 1 or from 2 to 4 linked groups independently selected from - [C(R~)(R2)]"-, -C(R~)=C(R2)-, -C(R~)=N, -C(=NREM)-, -C(=0)-, -C(=S)-, -0-, -Si(Ri)q-, -S(=0) -and - N(R&)-; wherein: x is 0, 1, or 2; n is 1, 2, 3, or 4; each R& and R2 is, independently, H, a protecting group, hydroxyl, C»C» alkyl, substituted C» (-CHz-) group connecting the 2' oxygen atom and the 4' carbon atom, for which the term methyleneoxy (4'-CH&-0-2') LNA is used.

Furthermore, in the case of the bicyclic sugar moiety having an ethylene bridging group in this position, the ethyleneoxy (4'-CH&CH&-0-2') LNA is used. n -L- methyleneoxy (4'-CH&-0-2'), an isomer of methyleneoxy (4'-CH&-0-2') LNA is also encompassed within the definition of LNA, as used herein.

In some embodiments, the nucleoside unit is an LNA unit selected from the list of beta-D-oxy-LNA, alpha-Loxy-LNA, beta-D-amino-LNA, alpha-L-amino-LNA, beta-D-thio-LNA, alpha-L-thio-LNA, 5'-methyl-LNA, beta-D-ENA and alpha-L-ENA.

"cEt" or "constrained ethyl" means a bicyclic sugar moiety comprising a bridge connecting the 4'-carbon and the 2'-carbon, wherein the bridge has the formula: 4'-CH(CHq)-0-2'.

"Constrained ethyl nucleoside" (also cEt nucleoside) means a nucleoside comprising a bicyclic sugar moiety comprising a 4'-CH(CH3)-0-2' bridge. cEt and some of its properties are described in Pallan et al. Chem Commun (Camb). 2012, August 25; 48(66): 8195-8197.

"Tricyclo (tc)-DNA" belongs to the class of conformationally constrained DNA analogs that show enhanced binding properties to DNA and RNA. Structure and method of production may be seen in Renneberg et al. Nucleic Acids Res. 2002 Jul 1; 30(13): 2751-2757.

"2'-fluoro", as referred to herein is a nucleoside comprising a fluoro group at the 2' position of the sugar ring. 2'-fluorinated nucleotides are described in Peng et al. J Fluor Chem. 2008 September; 129(9): 743-766.

"2'-O-methyl", as referred to herein, is a nucleoside comprising a sugar comprising an -OCH3 group at the 2' position of the sugar ring.

"Conformationally Restricted Nucleosides (CRN)" and methods for their synthesis, as referred to herein, are described in WO2013036868, which is hereby incorporated by reference. CRN are sugar-modified nucleosides, in which, similar to LNA, a chemical bridge connects the C2' and C4' carbons of the ribose. However, in a CRN, the C2' - C4' bridge is one carbon longer than in an LNA molecule. The chemical bridge in the ribose of a CRN locks the ribose in a fixed position, which in turn restricts the flexibility of the nucleobase and phosphate group. CRN substitution within an RNA- or DNA-based oligonucleotide has the advantages of increased hybridization affinity and enhanced resistance to nuclease degradation.

"Unlocked Nucleic Acid" or "UNA", is as referred to herein unlocked nucleic acid typically where the C2 - C3 C-C bond of the ribose has been removed, forming an unlocked "sugar" residue (see Fluiter et al., Mol. Biosyst., 2009, 10, 1039, hereby incorporated by reference, and Snead et al. Molecular Therapy-Nucleic Acids (2013) 2, e103;).

"Target region" means a portion of a target nucleic acid to which one or more antisense compounds is targeted.

"Targeted delivery" as used herein means delivery, wherein the antisense oligonucleotide has either been formulated in a way that will facilitate efficient delivery in specific tissues or cells, or wherein the antisense oligonucleotide in other ways has been for example modified to comprise a targeting moiety, or in other way has been modified in order to facilitate uptake in specific target cells.

In the context of the present invention, the term "targeting moiety" or "homing moiety" refers to a molecule or chemical group that is capable of directing or guiding the inhibitor/antisense oligonucleotide to a specific target tissue or organ. The moiety may be attached to the inhibitor/antisense oligonucleotide to enhance it targeting capabilities, allowing it to bind to a particular tissue or cell with increase specificity. The use of such homing moiety may improve efficacy of the inhibitor and/or reduce potential adverse side effects. Thus, when reference is made to a homing moiety targeted to the liver, it should be understood that the homing moiety is capable of directing or guiding the inhibitor/antisense oligonucleotide to or facilitating uptake in the liver.

### Brief descriptions of the figures

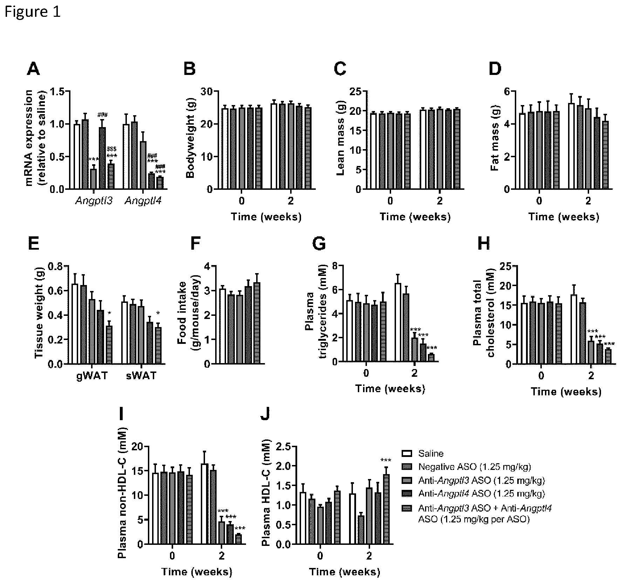

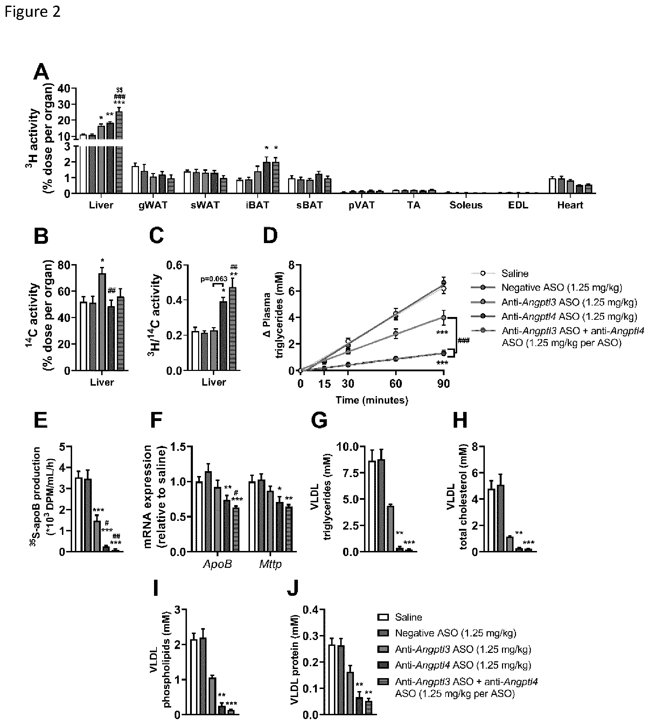

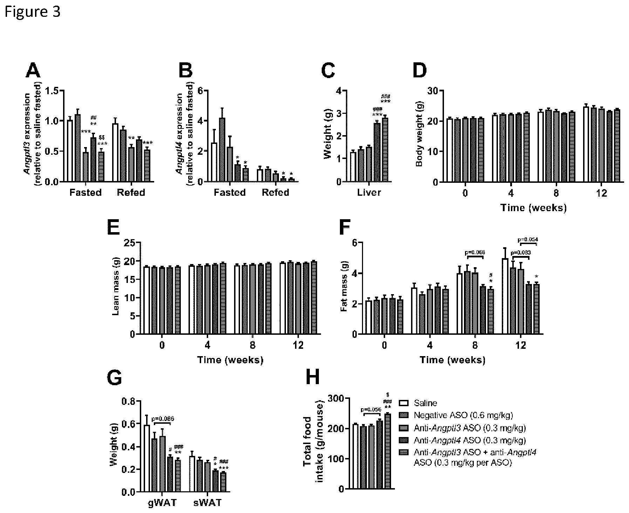

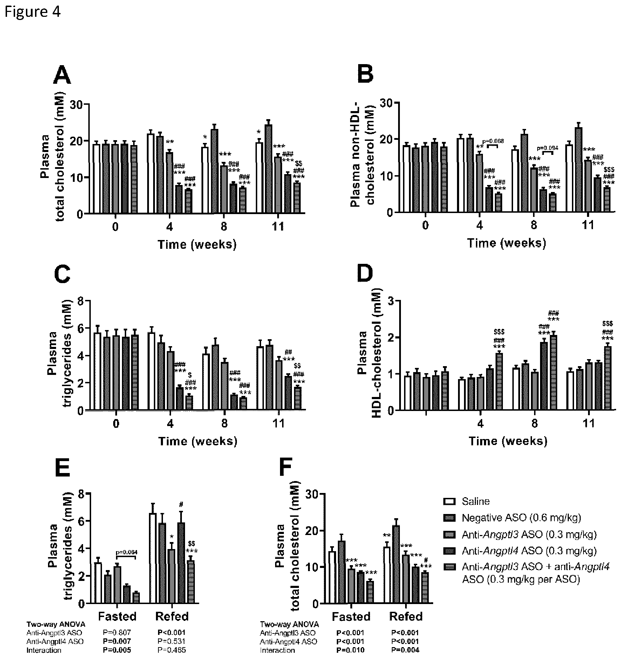

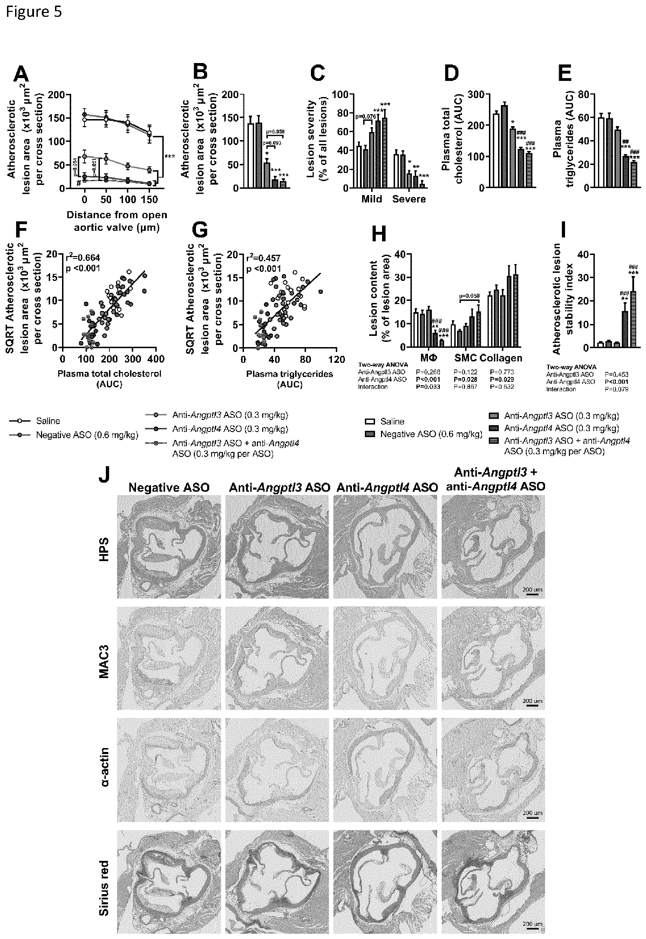

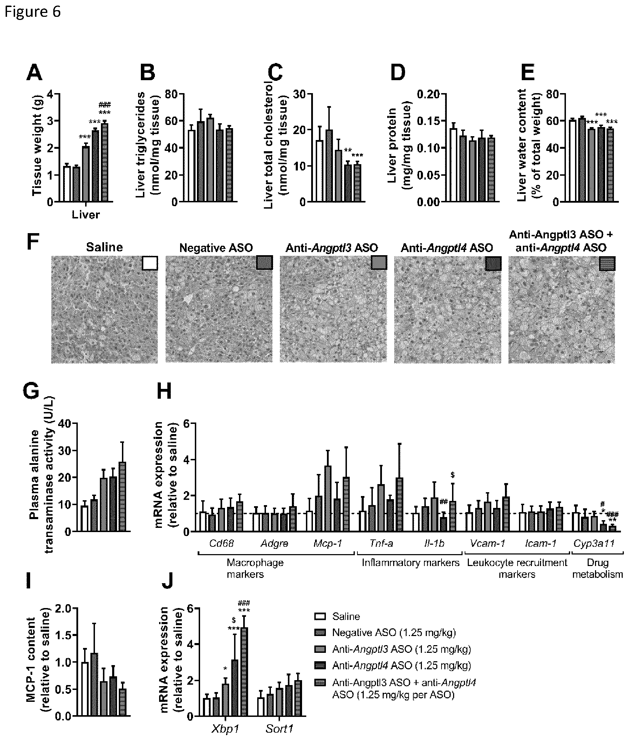

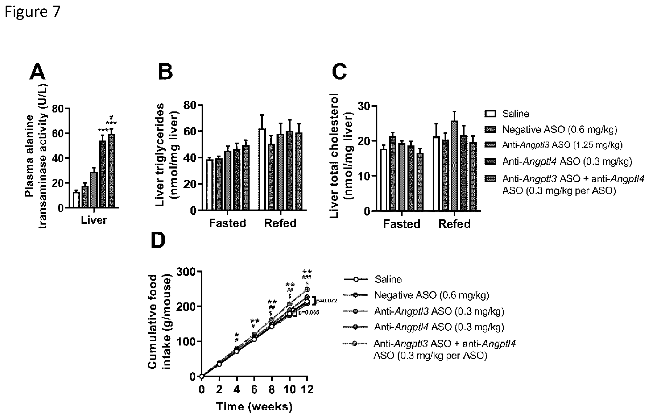

Figure 1. Short-term liver-targeted Angptl3 and Angptl4 silencing alleviates hyperlipidemia in APOE*3-Leiden.CETP mice. Over a period of two weeks, female APOE*3-Leiden.CETP mice were injected five times with saline (white) or the following liver-targeted antisense oligonucleotides (ASO): negative ASO (scrambled; 1.25 mg/kg, grey), anti-Angptl3 ASO (1.25 mg/kg, red), anti-Angptl4 ASO (1.25 mg/kg, blue), or anti-Angptl3 ASO + anti-Angptl4 ASO (1.25 mg/kg per ASO, red-blue striped). At the endpoint, hepatic (A) expression of Angptl3 and Angptl4 was measured (n=6-8 per group). At week 0 and 2, (B) body weight (C) lean mass, and (D) fat mass were determined (n=8 per group). (E) Gonadal white adipose tissue (gWAT) and subcutaneous (s)WAT were weighed (n=8 per group). During the last week of the experiment (F), food intake was monitored while mice were single housed (n=6 per group). At week 0 and 2, levels of plasma (G) triglycerides, (H) total cholesterol, (I) non-high-density lipoprotein (HDL)-cholesterol (J) and HDL-cholesterol were measured after 4 hours of fasting (n=7-8 per group). Values are presented as means ± SEM. * any group vs. negative ASO; # any group vs. anti-Angptl3 ASO; $ anti-Angptl4 ASO vs. anti-Angptl3 ASO + anti-Angptl4 ASO. ^{∗} p≤0.05, ^{∗∗} p≤0.01, ***,###,$$$ p≤0.001 according to one-way ANOVA (A, E, F), two-way ANOVA (B-D, G, H) or mixed effects analysis (I, J) and following Tukey's (A-H) or Šídák's (I, J) multiple-comparison test, respectively.
Figure 2. Liver-targeted Angptl4 silencing elevates lipid uptake by brown adipose tissue and liver while reducing hepatic VLDL production in APOE*3-Leiden.CETP mice. Over a period of two weeks, female APOE*3-Leiden.CETP mice were injected five times with saline (white) or the following liver-targeted antisense oligonucleotides (ASO): negative ASO (scrambled; 1.25 mg/kg, grey), anti-Angptl3 ASO (1.25 mg/kg, red), anti-Angptl4 ASO (1.25 mg/kg, blue), or anti-Angptl3 ASO + anti-Angptl4 ASO (1.25 mg/kg per ASO, red-blue striped). At the end of the treatment period, half of the mice was injected with triglyceride (TG)-rich lipoprotein-like particles double-labeled with glycerol tri[3H]oleate and [14C]cholesteryl oleate to assess uptake of (A) [3H]oleate and (B) [14C]cholesteryl oleate by liver, gonadal white adipose tissue (gWAT), subcutaneous (s)WAT, interscapular brown adipose tissue (iBAT), subscapular (s)BAT, perivascular (pV)AT, tibialis anterior (TA) muscle, soleus, extensor digitorum longus (EDL) and heart (n=7-8 per group). (C) The ratio of 3H-activity over 14C-activity is shown for the liver (n=7 per group). At the endpoint, the other half of the mice was injected with Tran[35S] label followed by Triton WR1339, and TG levels were determined in plasma samples drawn at the indicated time points, which were (D) plotted as the increase in TG to baseline, from which VLDL-TG production rate was determined by linear regression. (E) ApoB production was determined by measuring 35S in isolated VLDL (n=6-8 per group). Hepatic (F) gene expression of ApoB and Mttp was measured (n=6-8 per group). Concentrations of (G) TG, (H) total cholesterol, (I) phospholipids and (J) protein within VLDL particles were measured and corrected for the total volume of isolated VLDL (n=6-8 per group). Values are presented as means ± SEM. * any group vs. negative ASO; # any group vs. anti-Angptl3 ASO; $ anti-Angptl4 ASO vs. anti-Angptl3 ASO + anti-Angptl4 ASO *,# p<0.05; **,##,$$ p<0.01; ***,### p<0.001, according to one-way ANOVA (A-F) or Kruskal-Wallis test (G-J) and following Tukey's (A-F) or Dunn's (G-J) multiple-comparison test.
Figure 3. Long-term liver-targeted Angptl4 silencing lowers adiposity in APOE*3-Leiden.CETP mice. Over a period of 12 weeks, female APOE*3-Leiden.CETP mice were injected with saline (white) or the following liver-targeted antisense oligonucleotides (ASO): negative ASO (scrambled; 1.25 mg/kg, grey), anti-Angptl3 ASO (0.3 mg/kg, red), anti-Angptl4 ASO (0.3 mg/kg, blue), or anti-Angptl3 ASO + anti-Angptl4 ASO (0.3 mg/kg per ASO, red-blue striped). At the endpoint, hepatic expression of (A) Angptl3 and (B) Angptl4 were measured (n=7-8 per group) and (C) livers were weighed (n=16 per group). Every four weeks, (D) body weight, (E) lean mass and (F) fat mass were measured (n=15-16 per group). At the endpoint, (G) gonadal white adipose tissue (gWAT) and subcutaneous (s)WAT were weighed (n=15-16 per group). (H) Food intake was monitored and expressed as total per mouse (n=4 cages of three to four mice per cage). Values are presented as means ± SEM. * any group vs. negative ASO; # any group vs. anti-Angptl3 ASO; $ any group vs. anti-Angptl4. *,#,$ p<0.05; ** p<0.01; ***,### p<0.001, according to two-way ANOVA (A, B, D, E, H), one-way ANOVA (C), two-way ANOVA on ranked values (F), or Kruskal-Wallis test (G) and following, Šídák's (C), Tukey's (A, B, D, E, F, H), or Dunn's (G) multiple-comparison test, respectively.
Figure 4. Long-term liver-targeted Angptl3 and Angptl4 silencing lowers plasma TG in refed and fasted stare, respectively, in APOE*3-Leiden.CETP mice. Over a period of 12 weeks, female APOE*3-Leiden.CETP mice were injected with saline (white) or the following hepatocyte-targeted antisense oligonucleotides (ASO): negative ASO (scrambled; 1.25 mg/kg, grey), anti-Angptl3 ASO (0.3 mg/kg, red), anti-Angptl4 ASO (0.3 mg/kg, blue), or anti-Angptl3 ASO + anti-Angptl4 ASO (0.3 mg/kg per ASO, red-blue striped). Levels of plasma (A) total cholesterol, (B) non-high-density lipoprotein (HDL)-cholesterol, (C) triglycerides and (D) HDL-cholesterol were measured at week 0, 4, 8, and 11, after 4 hours of fasting (n=12-16 per group). At week 12, levels of plasma (E) triglycerides and (F), total cholesterol were measured after 16 hours of fasting (Fasted) or after 12 hours of fasting and 4 hours of refeeding (Refed) (n=7-8 per group). Values are presented as means ± SEM. * any group vs. negative ASO; # any group vs. anti-Angptl3 ASO; $ anti-Angptl4 ASO vs. anti-Angptl3 ASO + anti-Angptl4 ASO *,#,$ p<0.05; **,## p<0.01; ***,###,$$$ p<0.001, according to two-way ANOVA on ranked values (A, B, C,), two-way ANOVA (D, E, F) and following Tukey's multiple-comparison test. To discern anti-Angptl3 ASO and anti-Angptl4 ASO effects, extra two-way ANOVAs were performed per nutritional state for (E, F), in which saline was excluded; values are shown below graphs.
Figure 5. Long-term liver-targeted Angptl3 and Angptl4 silencing attenuates atherosclerosis development in APOE*3-Leiden.CETP mice. Over a period of 12 weeks, female APOE*3-Leiden.CETP mice were injected with saline (white) or the following hepatocyte-targeted antisense oligonucleotides (ASO): negative ASO (scrambled; 1.25 mg/kg, grey), anti-Angptl3 ASO (0.3 mg/kg, red), anti-Angptl4 ASO (0.3 mg/kg, blue), or anti-Angptl3 ASO + anti-Angptl4 ASO (0.3 mg/kg per ASO, red-blue striped), after which mice were killed and hearts were collected. Cross-sections of the aortic root area were stained with hematoxylin-phloxine-saffron, and (A) atherosclerotic lesion area was determined and expressed as a function of distance from the appearance of open aortic valves and (B) the mean atherosclerotic lesion area was calculated (n=15-16 per group). (C) Lesions were categorized according to lesion severity and expressed as a percentage of total lesions (n=12-16 per group). (D) Total cholesterol and (E) triglyceride exposure during the study were determined by calculating the area under the curve (AUC) and (F,G) plotted against the square root (SQRT) of the mean atherosclerotic lesion area from which B and Pearson correlation coefficients were determined (n=13-16 per group). Cross-sections of the aortic root area were stained with anti-MAC3 antibody, anti-α-actin antibody and Sirius Red to (H) quantify the content of macrophages (Mφ), smooth muscle cells (SMC), and collagen, respectively, within the lesions (n=15-16 per group). (I) Lesion stability index was calculated by dividing the sum of collagen and smooth muscle cell content by the macrophage content (n=15-16 per group). (H) Representative pictures are shown (n=15-16 per group). Values are presented as means ± SEM (n=12-16 per group). * any group vs. negative ASO; # any group vs. anti-Angptl3 ASO; *,# p<0.05; ** p<0.01; ***,### p<0.001 according to one-way ANOVA (A, D) or Kruskal-Wallis test (B, C, E, H, I) and following Šídák's (A, D) or Dunn's (B, C, E, H, I) multiple-comparison test respectively. To discern anti-Angptl3 ASO and anti-Angptl4 ASO effects, extra two-way ANOVAs were performed for (H, I), in which saline was exclude; values are shown below graphs.
Figure 6. Liver-targeted Angptl3 and Angptl4 silencing induces hypertrophy of hepatocytes without causing hepatic steatosis or inflammation in APOE*3-Leiden.CETP mice. Over a period of two weeks, female APOE*3-Leiden.CETP mice were injected five times with saline (white) or the following hepatocyte-targeted antisense oligonucleotides (ASO): negative ASO (scrambled; 1.25 mg/kg, grey), anti-Angptl3 ASO (1.25 mg/kg, red), anti-Angptl4 ASO (1.25 mg/kg, blue), or anti-Angptl3 ASO + anti-Angptl4 ASO (1.25 mg/kg per ASO, red-blue striped). At endpoint, (A) liver weight and hepatic content of (B) triglycerides, (C) total cholesterol, (D) protein and (E) water were determined (n=7-8 per group). (F) Liver cross-sections were stained with hematoxylin & eosin (H&E) (n=8 per group). In plasma, (G) alanine transaminase activity was determined (n=8 per group). Hepatic (H) gene expression was measured of macrophage markers [cluster of differentiation 68 (Cd68), adhesion G Protein-Coupled Receptor E1 (Adgre), monocyte chemoattractant protein-1 (Mcp-1)], inflammatory markers [tumor necrosis factor alpha (Tnf-a), interleukin-1b (II-1b)], leucocyte recruitment markers [vascular cell adhesion protein 1 (Vcam-1), intercellular Adhesion Molecule 1 (Icam-1)] and drug metabolism [cytochrome P450 3A11 (Cyp3a11)] (n=6-8 per group). (I) MCP-1 content was quantified by immunostaining (n=6-8 per group). Hepatic (J) gene expression was measured of endoplasmic reticulum (ER) stress markers [X-box binding protein 1 (Xbp1), sortilin 1 (Sort1)] (n=6-8 per group). Values are presented as means ± SEM. * any group vs. negative ASO; # any group vs. anti-Angptl3 ASO; $ anti-Angptl4 ASO vs. anti-Angptl3 ASO + anti-Angptl4 ASO *,#,$ p<0.05; **,## p<0.01; ***,### p<0.001, according to one-way ANOVA (A, E, H, J), Kruskal-Wallis test (B-D, G, I) and following Šídák's (A, E), Dunn's (B-D, G, I), or Tukey's (H, J) multiple-comparison test.
Figure 7. Long-term liver-targeted Angptl3 and Angptl4 silencing does not change hepatic lipid content in APOE*3-Leiden.CETP mice. Over a period of 12 weeks, female APOE*3-Leiden.CETP mice were injected with saline (white) or the following hepatocyte-targeted antisense oligonucleotides (ASO): negative ASO (scrambled; 1.25 mg/kg, grey), anti-Angptl3 ASO (0.3 mg/kg, red), anti-Angptl4 ASO (0.3 mg/kg, blue), or anti-Angptl3 ASO + anti-Angptl4 ASO (0.3 mg/kg per ASO, red-blue striped). In plasma of endpoint, (A) alanine transaminase activity was determined (n=13-16 per group). Hepatic content of (B) triglycerides and (C) total cholesterol were determined (n=14-16 per group). (D) Food intake was monitored and expressed as cumulative food intake per mouse (n=4 cages of three to four mice per cage). Values are presented as means ± SEM. * any group vs. negative ASO; # any group vs. anti-Angptl3 ASO; $ anti-Angptl4 ASO vs. anti-Angptl3 ASO + anti-Angptl4 ASO *,#,$ p<0.05; **,## p<0.01; ### p<0.001, according to Kruskal-Wallis test (A) or two-way ANOVA (B-D) and following Dunn's (A), Šídák's (B, C) orTukey's (D) multiple comparisons tests.

### Detailed description

The present invention provides novel compounds, compositions and uses of such, that are potent inhibitors of ANGPTL4. Such compounds are useful in compositions for medical use, such as for treatment, alleviation, amelioration, pre-emptive treatment or prophylaxis of diseases where modulation of ANGPTL4 is beneficial. Such diseases comprise cardiovascular disease, such as atherosclerosis, high level in circulation of total cholesterol, plasma triglycerides, triglyceride-rich lipoproteins, VLDL-ApoB or for treatment of low plasma HDL-C levels, such as for increasing plasma HDL-C levels. Further, the compounds and compositions of the invention are useful for treatment, alleviation, amelioration, pre-emptive treatment or prophylaxis of atherosclerotic lesions, for reducing atherosclerotic lesion size, for reducing the number of severe atherosclerotic lesions, or for improving the atherosclerotic lesion index in a patient, such as in a mamma, such as in a primate, such as in a human. Data in the experimental section support these uses, and the below embodiments. The compounds are useful for long term treatment,

Furthermore, the compounds and compositions of the invention are useful for long-term treatment of cardiovascular disease in mammals, such as in humans, such as treatment lasting more than 4 weeks.

The first aspect of the present invention relates to an ANGPTL4 inhibitor comprising an antisense oligonucleotide consisting of 10-50 nucleotides in length, wherein 10-30 consecutive nucleotides are complementary to SEQ ID NO.1 or SEQ ID NO. 2, and has at least one affinity-enhancing nucleotide analogue and comprises at least one phosphorothioate internucleoside linkage, and wherein the ANGPTL4 inhibitor is for use in treatment, alleviation, pre-emptive treatment, prevention or prophylaxis of a cardiovascular disease in a mammal.

The invention is based upon the surprising finding by the inventors that administration of an ANGPTL4 inhibitor according to the present invention or a combination of such an ANGPTL4 inhibitor combined with an ANGPTL3 inhibitor is capable of regulating disease parameters related to cardiovascular disease in a mammal, such as in a human, and thus the ANGPTL4 inhibitor according to the present invention is for use in treatment, alleviation, amelioration, pre-emptive treatment or prophylaxis of cardiovascular disease in a mammal such as a human. In some embodiments, the cardiovascular disease is atherosclerosis.

In some embodiments, the use is for lowering of plasma triglycerides and/or total cholesterol, such as for treatment of high levels of plasma triglycerides and/or high levels of total cholesterol. In some embodiments, the use is for reducing circulating triglyceride-rich lipoproteins, such as for treatment of high levels of circulating triglyceride-rich lipoproteins.

Surprisingly, the inventors found that the ANGPTL4 inhibitors of the invention are capable of increasing plasma HDL-C levels. In some embodiments, the use is for increasing plasma HDL-C levels, such as for treatment of low plasma HDL-C levels.

In some embodiments, the use is for reducing VLDL-ApoB production, such as for treatment of high VLDL-ApoB levels.

In some embodiments, the use is for prevention of atherosclerotic lesions. In some embodiments, the use is for reducing atherosclerotic lesion size. In some embodiments, the use is for reducing the number of severe atherosclerotic lesions. In some embodiments, the use is for improving the atherosclerotic lesion index in a patient.

In some embodiments, the inhibitor inhibits the expression of ANGPTL4 in a mammal such as a human.

In some embodiments, the inhibitor comprises an antisense oligonucleotide which is complementary to at least 10, such as at least 11, 12, 13, 14, 15, 16, 17, 18, 19 or 20 consecutive nucleotides of any one of position 1732 to 1759, from position 234-261, from position 1264-1296 of SEQ ID NO. 1 and/or from position 2800-2872, from position 3415-3442, from position 4968-4994 of SEQ ID NO.2 or a combination thereof. In some embodiments, the inhibitor comprises an antisense oligonucleotide which is complementary to at least 10, such as at least 11, 12, 13, 14, 15, 16, 17, 18, 19 or 20 consecutive nucleotides of 1264-1295, such as is fully complementary to nucleotides 1269-1295 of SEQ ID NO. 1.

In some embodiments, the modified nucleotide is selected from the group consisting of a bridged nucleic acid such as LNA, cET, ENA, BNA, UNA, 2'Fluoro modified nucleotide, 2'0-Methyl modified nucleotide, 2'-Methoxyethyl modified nucleotide and a combination thereof. In some embodiments, the inhibitor comprises a gapmer antisense oligonucleotide having a gap of at least 5 consecutive DNA nucleotides

In some embodiments, the angptl4 comprises a sequence selected from the group consisting of SEQ ID N0.47, SEQ ID NO.159, SEQ ID NO.161, SEQ ID NO.163, SEQ ID NO.165, SEQ ID NO.173, SEQ ID NO.179, SEQ ID NO.186, SEQ ID NO.187, SEQ ID NO.3, SEQ ID NO.4, SEQ ID NO.5, SEQ ID NO.6, SEQ ID NO.7, SEQ ID NO.8, SEQ ID NO.9, SEQ ID NO.10, SEQ ID NO.11, SEQ ID NO.12, SEQ ID NO.13, SEQ ID NO.14, SEQ ID NO.15, SEQ ID NO.16, SEQ ID NO.17, SEQ ID NO.18, SEQ ID NO.19, SEQ ID NO.20, SEQ ID NO.21, SEQ ID NO.22, SEQ ID NO.23, SEQ ID NO.24, SEQ ID NO.25, SEQ ID NO.26, SEQ ID NO.27, SEQ ID NO.28, SEQ ID NO.29, SEQ ID NO.30, SEQ ID NO.31, SEQ ID NO.32, SEQ ID NO.33, SEQ ID NO.34, SEQ ID NO.35, SEQ ID NO.36, SEQ ID NO.37, SEQ ID NO.38, SEQ ID NO.39, SEQ ID NO.40, SEQ ID NO.41, SEQ ID NO.42, SEQ ID NO.43, SEQ ID NO.44, SEQ ID NO.45, SEQ ID NO.46, SEQ ID NO.47, SEQ ID NO.48, SEQ ID NO.49, SEQ ID NO.50, SEQ ID NO.51, SEQ ID NO.52, SEQ ID NO.53, SEQ ID NO.54, SEQ ID NO.55, SEQ ID NO.56, and or a combination of any of those.

In some embodiments, the antisense oligonucleotide is selected from the group consisting of

| | |
|---|---|
| +C*+A*+A*+T*G*T*G*A*C*T*G*A*G*T*+C*+C*+G | SEQIDNO.47, |
| +C*+A*+A*T*G*T*G*A*C*T*G*A*G*T*+C*+C*+G | SEQIDNO.47, |
| +C*+A*+A*T*G*T*G*A*C*T*G*A*G*+T*+C*+C*+G | SEQIDNO.47, |
| +T*+C*+G*A*G*A*T*G*A*A*C*G*G*+A*+G*+A | SEQIDNO.159, |
| +A*+A*+C*T*T*A*G*A*G*A*A*C*C*G*+C*+G*+A | SEQIDNO.161, |
| +T*+T*+A*G*A*G*A*A*C*C*G*C*G*+A*+G*+T | SEQIDNO.163, |
| +T*+C*+G*A*A*A*T*G*A*G*T*C*T*G*+C*+A*+C | SEQIDNO.165, |
| +C*+T*+T*A*A*C*A*G*T*G*G*A*T*G*+A*+C*+C | SEQIDNO.173, |
| +T*+A*+G*C*A*C*G*G*C*G*G*T*G*+G*+C*+G | SEQIDNO.179, |
| +A*+G*+T*A*C*T*G*G*C*C*G*T*T*G*+A*+G*+G | SEQIDNO.186, |
| +G*+A*+A*G*T*A*C*T*G*G*C*C*G*T*+T*+G*+A | SEQIDNO.187, |
| +A*G*G*C*T*A*A*G*T*T*A*G*T*G*G*+C*+C | SEQIDNO.3, |
| +G*+C*C*A*G*A*T*A*G*G*C*T*A*A*+G*+T*+T | SEQIDNO.4, |
| +C*+T*G*+G*A*A*A*G*A*A*T*C*G*G*+A*+T*+C | SEQIDNO.5, |
| +C*G*+C*T*G*G*A*A*A*G*A*A*T*+C*+G*+G*+A | SEQIDNO.6, |
| +G*C*+T*A*G*T*C*T*C*G*A*C*A*G*C*+A*+G | SEQIDNO.7, |
| +G*+A*T*C*C*T*C*A*T*G*A*T*A*G*G*+C*+C | SEQIDNO.8, |
| +G*+G*C*C*G*T*C*G*G*A*G*C*A*C*C*G*+C | SEQIDNO.9 |
| +C*+A*+T*G*C*T*G*T*G*G*T*T*C*G*+A*+G*+A | SEQIDNO.10, |
| +C*+G*T*G*C*G*C*C*A*G*G*A*C*A*+T*+T*+C | SEQIDNO.11, |
| +C*+G*+T*G*C*G*C*C*A*G*G*A*C*+A*+T*+T | SEQIDNO.12, |
| +T*C*C*G*T*G*C*G*C*C*A*G*G*A*+C*A*+T | SEQIDNO.13, |
| +G*G*A*G*T*C*C*G*T*G*C*G*C*C*+A*G*+G | SEQIDNO.14, |
| +T*+G*C*G*C*T*C*C*G*C*G*T*G*T*T*+C*+G | SEQIDNO15 |
| +T*+G*C*G*C*T*C*C*G*C*G*T*G*T*+T*+C | SEQIDNO.16, |
| +G*+T*G*C*G*C*T*C*C*G*C*G*T*G*T*+T | SEQIDNO.17, |
| +G*+T*+C*G*A*A*A*T*G*A*G*T*C*T*+G*+C*+A | SEQIDNO.18, |
| +G*+C*+A*C*G*T*C*T*G*G*A*G*G*C*+T*+C*+A | SEQIDNO.19, |
| +T*+T*+G*A*G*C*A*C*G*T*C*T*G*G*+A*+G*+G | SEQIDNO.20, |
| +G*+G*+A*T*C*G*C*A*G*T*G*C*C*G*+C*+A*+A | SEQIDNO.21, |
| +G*C*+T*G*A*A*T*T*C*G*C*A*G*G*+T*G*+C | SEQIDNO.23, |
| +G*+A*+T*C*G*C*A*G*T*G*C*C*G*+C*+A*+A | SEQIDNO.24, |
| +G*+T*+G*T*T*G*T*A*A*C*C*T*C*T*+T*+G*+T | SEQIDNO.25, |
| +C*+C*+G*T*G*T*T*G*T*A*A*C*C*T*+C*+T*+T | SEQIDNO.26, |
| +C*+C*+T*+C*A*T*G*G*T*C*T*A*G*G*+T*+G*+C | SEQIDNO.27, |
| +C*A*+C*C*T*C*A*T*G*G*T*C*T*A*G*+G*+T | SEQIDNO.28, |
| +G*G*C*G*C*C*T*C*T*G*A*A*T*T*+A*+C*+T | SEQIDNO.29, |
| +C*+G*+T*G*G*C*G*C*C*T*C*T*G*A*+A*+T*+T | SEQIDNO.30, |
| +T*C*G*T*G*G*C*G*C*C*T*C*T*G*A*+A*+T | SEQIDNO.31, |
| +C*+C*+G*C*C*T*T*G*T*A*G*G*C*T*T*C*+C | SEQIDNO.32, |
| +A*+C*+T*C*G*G*C*G*T*T*G*C*C*A*T*C*+C | SEQIDNO.33, |
| +G*+C*C*G*T*G*T*C*C*T*C*G*C*C*A*C*+C | SEQIDNO.34, |
| +G*+A*G*G*T*C*G*T*G*A*T*C*C*T*G*+G*+T | SEQIDNO.35, |
| +G*+C*+G*G*A*G*G*T*C*G*T*G*A*T*C*+C*+T | SEQIDNO.36, |
| +C*C*T*G*C*G*G*A*G*G*T*C*G*T*+G*+A*+T | SEQIDNO.37, |
| +C*+T*+C*T*T*G*G*C*G*C*A*G*T*T*+C*+T*+T | SEQIDNO.38, |
| +G*+C*+T*C*T*T*G*G*C*G*C*A*G*T*+T*+C*+T | SEQIDNO.39, |
| +G*+G*+C*T*C*T*T*G*G*C*G*C*A*G*T*+T*+C | SEQIDNO.40, |
| +G*+G*T*G*C*C*A*A*A*C*C*A*C*C*+A*+G*+C | SEQIDNO.41, |
| +G*+G*+A*+G*C*G*G*A*A*G*T*A*C*T*G*+G*+C | SEQIDNO.42, |
| +A*+T*+G*G*A*G*C*G*G*A*A*G*T*+A*+C*+T*+G | SEQIDNO.43, |
| +G*+G*+A*T*G*G*A*G*C*G*G*A*A*G*+T*+A*+C | SEQIDNO.44, |
| +G*+G*C*T*G*G*A*T*C*A*A*C*A*T*+G*+G*+T | SEQIDNO.45, |
| +A*+T*+G*T*G*A*C*T*G*A*G*T*C*C*+G*+C*+C | SEQIDNO.46, |
| +G*+A*+A*C*T*C*T*G*T*G*A*G*C*T*C*C*+G | SEQIDNO.48, |
| +C*+T*+G*G*T*G*G*A*C*T*A*A*C*A*+C*+A*+C | SEQIDNO.49, |
| +T*+G*+G*A*C*C*T*G*A*C*A*A*G*G*+A*+G*+A | SEQIDNO.50, |
| +C*+A*+C*A*C*G*G*C*A*T*G*T*A*+A*+G*+G | SEQIDNO.51, |
| +G*+C*+C*A*G*A*T*A*G*G*C*T*A*A*+G*+T*+T | SEQIDNO.4, |
| +A*+G*+G*C*T*A*G*T*C*T*C*G*A*C*+A*+G*+C | SEQIDNO.52, |
| +G*+A*+T*C*C*T*C*A*T*G*A*T*A*G*+G*+C*+C | SEQIDNO.8, |
| +G*+A*+T*C*G*C*A*G*T*G*C*C*G*C*+A*+A*+T | SEQIDNO.53, |
| +G*A*T*C*G*C*A*G*T*G*C*C*G*C*+A*+A*+T | SEQIDNO.53, |
| +C*+G*+T*G*T*T*G*T*A*A*C*C*T*C*+T*+T*+G | SEQIDNO.54, |
| +G*+A*+T*C*G*C*A*G*T*G*C*C*+G*+C*+A | SEQIDNO.55, |
| +G*+A*+T*C*G*C*A*G*T*G*C*C*G*+C*+A | SEQIDNO.55, |
| +G*+G*+A*T*C*G*C*A*G*T*G*C*+C*+G*+C | SEQIDNO.56, |
| +C*+T*+G*G*A*A*A*G*A*A*T*C*G*G*+A*+T*+C | SEQIDNO.5, |

wherein + are LNA, * are phosphorothioate internucleotide linkages, and optionally C is methyl-Cytosine.

In some embodiments, the LNA is beta-D-oxy LNA .

The inventors have now shown that an ANGPTL4 compound according to invention, which is targeted to the liver is not toxic, and is capable of regulating important disease parameters relating to cardiovascular disease, such as atherosclerosis.

In some embodiments, the inhibitor is coupled to a homing moiety that target the liver.

In some embodiments, the homing moiety is a GalNac moiety.

In some embodiments, the GalNac moiety is a triantennary GalNac moiety.

In some embodiments, the homing moiety is attached to the inhibitor by a linker.

In some embodiments, the linker is one of a nucleotide linker or an amino linker.

In some embodiments, the linker is a nucleotide linker with a length of between 1 and 20 nucleotides.

In some embodiments, the linker is an amino linker with a length of between NH-(CH2)2-12.

In some embodiments, the linker is a NH-(CH2)6 or a NH-(CH2)8 or a NH-(CH2)10 linker.

In some embodiments, the linker and triantennary GalNac homing moiety has the structure

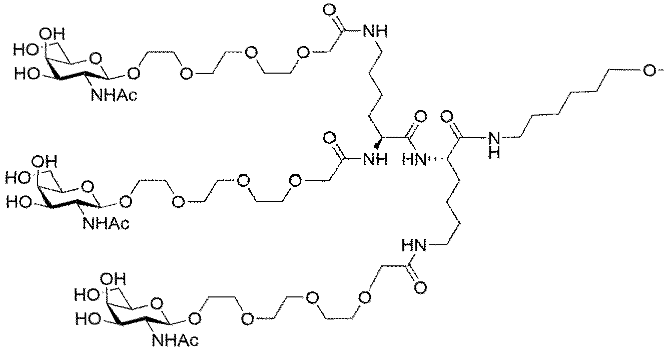

The inventors of the present invention have shown in the examples that the use of a combination of an ANGPTL4 and an ANGPTL3 inhibitor is capable of e.g. lowering plasma triglycerides and cholesterol and VLDL-ApoB.

Thus, in some embodiments, the ANGPTL4 inhibitor is for use in combination with an ANGPTL3 inhibitor. In some embodiments, the ANGPTL4 inhibitor is for use in combination with an ANGPTL3 inhibitor in a method for lowering plasma triglycerides, cholesterol and/or VLDL-ApoB.

ANGPTL3 expression may be inhibited by e.g. an ANGPTL3 antibody or by an ANGPTL3 antisense oligonucleotide, thus in some embodiments, the ANGPTL3 inhibitor is an ANGPTL3 antibody and/or an ANGPTL3 antisense oligonucleotide.

The a second aspect of this disclosure the invention relates to the finding by the inventors that targeting an ANGPTL4 inhibitor to the liver removes the expected toxicity but still cause significant regulation of relevant cardiovascular disease parameters. Thus, the second aspect relates to an ANGPTL4 inhibitor comprising the antisense oligonucleotide with the sequence +A*+G*+T*A*C*T*G*G*C*C*G*T*T*G*+A*+G*+G SEQ ID NO.186, wherein + are LNA such as beta-deoxy LNA, * are phosphorothioate internucleotide linkages, C is methyl-Cytocine, and wherein the inhibitor is attached to a homing moiety targeted to the liver.

Various liver homing moieties suitable for homing an antisense oligonucleotide to liver cells exist, and are as such embraced by the present invention.

In some embodiments, the homing moiety is a GalNac molecule. In some embodiments, the GalNac molecule is a tri-antennary GalNac. In some embodiments, the homing moiety is attached to the inhibitor by a linker. A variety of linkers exist, which are useful in attaching antisense oligonucleotides to homing moieties. Those are all embraced by the present invention. In some embodiments, the linker is one of a nucleotide linker or an amino linker. In some embodiments, the linker is a nucleotide linker with a length of between 1 and 20 nucleotides. In some embodiments, the linker is an amino linker with the formula NH-(CH2)2-12. In some embodiments, the linker is a NH-(CH2)6, or NH-(CH2)8 or NH-(CH2)10 linker.

In some embodiments, the angptl4 compound comprises the antisense oligonucleotide with the sequence +A*+G*+T*A*C*T*G*G*C*C*G*T*T*G*+A*+G*+G SEQ ID NO. 186, wherein + are LNA such as beta-deoxy LNA, * are phosphorothioate internucleotide linkages, C is methyl-Cytocine, and wherein the inhibitor is attached to a triantennary GalNac moiety targeted to the liver, and a linker with formula NH-(CH2)6.

In some embodiments, the ANGPTL4 compound consist of the antisense oligonucleotide with the sequence +A*+G*+T*A*C*T*G*G*C*C*G*T*T*G*+A*+G*+G SEQ ID NO. 186, wherein + are LNA such as beta-deoxy LNA, * are phosphorothioate internucleotide linkages, C is methyl-Cytocine, and wherein the inhibitor is attached to a triantennary GalNac moiety targeted to the liver, and a linker with formula NH-(CH2)6.

In some embodiments, the ANGPTL4 compound consist of the antisense oligonucleotide having the sequence +A*+G*+T*A*C*T*G*G*C*C*G*T*T*G*+A*+G*+G SEQIDNO.186, wherein + are LNA such as beta-deoxy LNA, * are phosphorothioate internucleotide linkages, C is methyl-Cytocine, and wherein the inhibitor is attached to a liver targeting homing moiety wherein linker and homing moiety has the structure

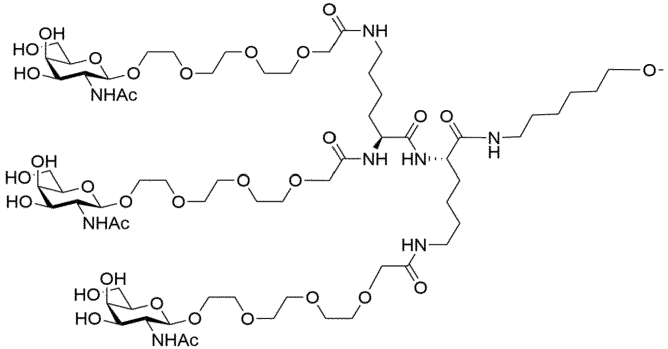

The inventors of the present invention has shown some superior effects of the compound of the second aspect of the invention, which shows a clear value of the compound for use as a medicament, in particular for use in treating cardiovascular disease, and for regulating cardiovascular disease parameters.

In some embodiments, the compound according to the previous embodiments of the second aspect of the invention, are for use as a medicament. In some embodiments, the ANGPTL4 inhibitor according to the second aspect of the invention is comprised in a pharmaceutical composition. In some embodiments, the ANGPTL4 inhibitor according to the second aspect or a pharmaceutical composition comprising this compound is for use in a method of treatment.

In some embodiments, the inhibitor according to the second aspect is for use in treatment, alleviation, pre-emptive treatment, prevention or prophylaxis of a cardiovascular disease in a mammal. In some embodiments, the mammal is a human.

In some embodiments, the cardiovascular disease is atherosclerosis. In some embodiments, the use is for lowering of plasma triglycerides and/or total cholesterol, such as for treatment of high levels of plasma triglycerides and/or high levels of total cholesterol. In some embodiments, the use is for reducing circulating triglyceride-rich lipoproteins, such as for treatment of high levels of circulating triglyceride-rich lipoproteins. In some embodiments, the use is for increasing plasma HDL-C levels, such as for treatment of low plasma HDL-C levels. In some embodiments, the use is for reducing VLDL-ApoB production, such as for treatment of high VLDL-ApoB levels.

The inventors have shown in the experimental part that the ANGPTL4 compounds according to the invention or the ANGPTL4 compounds in combination with an ANGPTL3 inhibitor, are safe when administered to a mammal, and that they are able to influence the development of atherosclerosis as measured in various parameters, such as preventing atherosclerotic lesions, reducing atherosclerotic lesion size, reducing the number of severe atherosclerotic lesions, or improving the atherosclerotic lesion index in a patient.

In some embodiments, the use is for prevention of atherosclerotic lesions. In some embodiments, the use is for reducing atherosclerotic lesion size. In some embodiments, the use is for reducing the number of severe atherosclerotic lesions. In some embodiments, the use is for improving the atherosclerotic lesion index in a patient.

The present invention further provides methods of treatment, alleviation, pre-emptive treatment, prevention or prophylaxis of a cardiovascular disease in a mammal (such as a human); said method comprising administrating a therapeutic effective amount of the ANGPTL4 inhibitor of the invention. In one embodiment, the cardiovascular disease is atherosclerosis. The present invention further provides methods for lowering of plasma triglycerides and/or total cholesterol, such as for treatment of high levels of plasma triglycerides and/or high levels of total cholesterol, said method comprising administrating a therapeutic effective amount of the ANGPTL4 inhibitor of the invention. The present invention provides methods for reducing circulating triglyceride-rich lipoproteins, such as for treatment of high levels of circulating triglyceride-rich lipoproteins, said method comprising administrating a therapeutic effective amount of the ANGPTL4 inhibitor of the invention. The present invention provides methods for increasing plasma HDL-C levels, such as for treatment of low plasma HDL-C levels, said method comprising administrating a therapeutic effective amount of the ANGPTL4 inhibitor of the invention. The present invention provides methods for reducing VLDL-ApoB production, such as for treatment of high VLDL-ApoB levels, said method comprising administrating a therapeutic effective amount of the ANGPTL4 inhibitor of the invention. The present invention provides methods for preventing or reducing the risk of atherosclerotic lesions, said method comprising administrating a therapeutic effective amount of the ANGPTL4 inhibitor of the invention. The present invention provides methods for reducing atherosclerotic lesion size, said method comprising administrating a therapeutic effective amount of the ANGPTL4 inhibitor of the invention. The present invention provides methods for reducing the number of atherosclerotic lesions (such as severe atherosclerotic lesions), said method comprising administrating a therapeutic effective amount of the ANGPTL4 inhibitor of the invention. The present invention provides methods for improving the atherosclerotic lesion index in a subject, said method comprising administrating a therapeutic effective amount of the ANGPTL4 inhibitor of the invention.

In some embodiments, the inhibitor inhibits the expression of ANGPTL4 mRNA. In some embodiments, the inhibitor is for use in combination with another drug. In some embodiments, the inhibitor is for use in combination with an ANGPTL3 inhibitor. In some embodiments, the ANGPTL3 inhibitor is an antisense oligonucleotide or an antibody against ANGPTL3. In some embodiments, the inventors have found that the ANGPTL4 inhibitor according to any of the above embodiments is for use in a pharmaceutical composition made for repeated administration to a primate, such as a human, for a period of at least 4 weeks. The inventors surprisingly found that even a long-term administration of the ANGPTL4 inhibitors of the invention gave no toxic effects on the liver, when administered to primates. The inventors also found that ANGPTL4 inhibitors of the invention when administered to mice are capable of maintaining a continued effect when used alone or e.g. in combination with an ANGPTL3 inhibitor, which is in contrast to the inventors finding that ANGPTL3 inhibitors when used alone loses effect when used longer than 4 weeks.

Accordingly, in some embodiments the invention relates to an ANGPTL4 inhibitor, or an ANGPTL4 inhibitor in combination with an ANGPTL3 inhibitor according to the previous embodiments for use in a pharmaceutical composition, which is made for administration with the purpose of maintaining a continuous effective dosage in a primate such as in a human for a period of at least 4 weeks, such as at least 5 weeks such as at least any one of 6, 7, 8, 9, 10 weeks, such as at least 20 weeks or at least 25 weeks or at least 50 weeks.

The ANGPTL4 inhibitor according to the previous embodiment, wherein the effective dosage is maintained for as long as needed. The inventors has found no toxicological effects or loss of effect by long term use of the inhibitor in primates, why the inhibitor and compositions comprising the inhibitor is for use for as long as there is a need to reduce any of the clinical endpoints.

The ANGPTL4 inhibitor according to the invention, wherein the inhibitor is for lowering plasma Total Cholesterol, non-HDL-C or plasma Triglyceride levels. The ANGPTL4 inhibitor according to the previous embodiment, wherein the inhibitor is for use in combination with an ANGPTL3 inhibitor. The inventors found that when combining an ANGPTL4 inhibitor according to the invention, with an ANGPTL3 inhibitor of the invention, an improved effect is observed on long term administration of the compositions.

The person skilled in the art realizes that the present disclosure is not limited to the preferred embodiments described above. The person skilled in the art further realizes that modifications and variations are possible within the scope of the appended claims. Additionally, variations to the disclosed embodiments can be understood and effected by the skilled person in practicing the claimed disclosure, from a study of the drawings, the disclosure, and the appended claims.

### Dosages

The expression "effective dosage" denotes the dose of a drug that will achieve the desired effect. In the context of the present invention, the desired effect is lowering of the activity of ANGPTL4. Lowering of the activity of ANGPTL4 can be measured by either measuring the level of ANGPTL4, for example when using antisense oligonucleotides, such as the gapmer design antisense oligonucleotides which result in degradation of ANGPTL4 RNA or ANGPTL4 mRNA. ANGPTL4 inhibition may be measured directly or indirectly via secondary indicators of ANGPTL4 activity.

The compounds of the invention are for use in effective dosages, and the compositions comprise effective dosages of the compounds of the invention.

In some embodiments, the dosage of the compound administered at each dosing, such as unit dose, is within the range of 0.0001 mg/kg - 25 mg/kg.

In some embodiments, the effective dose is a dose that is sufficient to down-regulate ANGPTL4 mRNA, to a significant level over the time period between successive administration dosages, such as a level which is a therapeutic benefit to the subject.

The pharmaceutical compositions of the invention may in some embodiments be made for administration to provide for an initial dosage build up phase, which may, depending on the disease pathology, be followed by a maintenance dosage scheme for the purpose of maintaining a concentration of the compound in the subject, such as in a target tissue of the subject, which will be effective in the treatment of the disease. The effectiveness of the dosages may in example be measured by observation of a disease parameter indicative of the state of the disease, or may depending on the target tissue, be measurable by observation of various measurable disease state parameters in plasma, such as anyone of total cholesterol, plasma triglycerides, triglyceride-rich lipoproteins, VLDL-ApoB or plasma HDL-C levels.

### Pharmaceutical compositions

The present invention also provides pharmaceutical compositions. Such compositions may comprise a therapeutically effective amount of the therapeutic, and a pharmaceutically acceptable carrier. The term "pharmaceutically acceptable" may be defined as approved by a regulatory agency. The regulatory agency may for example be the European Medicines Agency, a Federal or a state government or listed in the U.S. Pharmacopeia or other generally recognized pharmacopeia for use in animals, and more particularly in humans. The term "therapeutically effective amount" may be defined as an amount of therapeutic which results in a clinically significant inhibition, amelioration or reversal of development or occurrence of a disorder or disease. The term "carrier" may refer to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water may be a preferred carrier when the pharmaceutical composition is administered intravenously. Saline solutions and aqueous dextrose and glycerol solutions may also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The composition, if desired, may also contain wetting or emulsifying agents, or pH buffering agents. These compositions may take the form of solutions, suspensions, emulsion, tablets, pills, capsules, powders, sustained-release formulations and the like. The composition may be formulated as a suppository, with traditional binders and carriers such as triglycerides. Oral formulation may include standard carriers such as pharmaceutical grades of mannitol, lactose, starch, magnesium stearate, sodium saccharine, cellulose, magnesium carbonate, etc. Such compositions may contain a therapeutically effective amount of the therapeutic, preferably in purified form, together with a suitable amount of carrier so as to provide the form for proper administration to the patient. The formulation may suit the mode of administration. Compositions for intravenous administration may be solutions in sterile isotonic aqueous buffer. Where necessary, the composition may also include a solubilizing agent and a local anaesthetic such as lignocaine to ease pain at the site of the injection. The ingredients may be supplied either separately or mixed together in unit dosage form, for example, as a dry lyophilized powder or water free concentrate in a hermetically sealed container such as an ampoule or sachette indicating the quantity of active agent. Where the composition is to be administered by infusion, it may be dispensed with an infusion bottle containing sterile pharmaceutical grade water or saline. Where the composition is administered by injection, an ampoule of sterile water for injection or saline may be provided so that the ingredients may be mixed prior to administration.

### Drug delivery

The compounds and compositions of the present invention are targeted to the liver. In some embodiments this is by attachment of a liver homing moiety such as a GalNac molecule, i.e. a triatennary GalNac. In some embodiments, the triantennary GalNac is attached using an amino linker such as a NH-(CH2)6 or a NH-(CH2)8 or a NH-(CH2)10 linker, and in some preferred embodiments, the linker and GalNac moiety has the formula

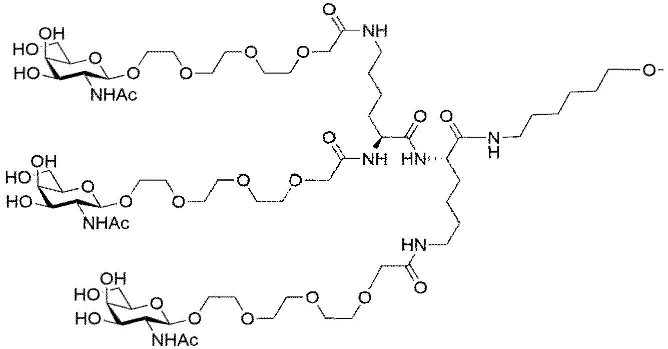

### Examples

### Synthesis and purification of antisense oligonucleotides

The synthesis and purification of antisense oligonucleotides is known in the art, and antisense oligonucleotides comprising modified nucleotides are commercially available. Specific design of antisense oligonucleotides and e.g. selection of nucleotide analogues, their amount and position in the antisense oligonucleotide, as well as selection of delivery moiety and linker determine their potency, and risk of potential side effects.

### Mouse experiments

APOE*3-Leiden.CETP mice were generated as previously described [12-15]. Female APOE*3-Leiden.CETP mice of 8-14 weeks of age were housed under standard conditions in groups of three to four mice per cage in a 12:12 hour light/dark cycle with *ad libitum* access to Western-type diet (16% fat, 0.15% cholesterol; Diet T; Ssniff-Spezialdiäten GmbH, Soest, Germany) and water unless stated otherwise. After a dietary run-in period of three weeks, baseline measurements were taken and mice were block-randomized into five intervention groups that were balanced for 4-hour fasted plasma TG and total plasma cholesterol (TC), age, body weight, and lean and fat body mass (EchoMR! 100-Analyzer; EchoMRI, Houston, Texas).

In experiment 1, mice (n=16 per group) were subcutaneously injected twice per week with saline, a liver-targeted negative (scrambled) ASO (1.25 mg/kg), anti-Angptl3 ASO (1.25 mg/kg), anti-Angptl4 ASO (1.25 mg/kg), or anti-Angptl3 ASO + anti-Angptl4 ASO (1.25 mg/kg per ASO) that were provided by Lipigon Pharmaceuticals AB (Umeå, Sweden). After two weeks, body weight, body composition, and food intake were measured. Then, mice were fasted for 4 hours before assessing plasma lipid levels as well as organ uptake of TRL-mimicking particles (n=8 per group) or hepatic very-low-density lipoprotein (VLDL) production (n=8 per group) around *Zeitgeber* time (ZT)6 . Livers from the mice in which organ uptake of lipids was assessed were used for gene expression analyses, histology, and lipid and water content measurements (n=8 per group).

In experiment 2, mice (n=16 per group) were subcutaneously injected with saline, a liver-targeted negative (scrambled) ASO (0.60 mg/kg), anti-*Angptl3* ASO (0.30 mg/kg), anti-*Angptl4* ASO (0.30 mg/kg), or anti-Angptl3 ASO + anti-*Angptl*4 ASO (0.30 mg/kg per ASO) twice per week for a total of 12 weeks. Body weight, body composition, food intake, and 4-hour fasted plasma lipid levels were monitored throughout the intervention. At the end of the study, eight mice per group were killed after 16 hours of fasting and the other eight mice per group after 12 hours of fasting followed by 4 hours of refeeding to measure plasma lipid levels in different nutritional states. All animal experiments were conducted in accordance with the Institute for Laboratory Animal Research Guide for the Care and Use of Laboratory Animals and were approved by the National Committee for Animal Experiments and by the Ethics Committee on Animal Care and Experimentation of the Leiden University Medical Center, The Netherlands.

### Gene expression analysis

RNA was isolated from frozen liver (approx. 30 mg) using TriPure RNA Isolation Reagent (11667165001, Sigma-Aldrich, Saint Louis, USA) and a FastPrep-24^{™} 5G bead beating grinder and lysis system (4.0 m/s for 10 sec; MP Biomedicals^{™}, Santa Ana, California, USA). cDNA was synthesized from 1 µg RNA using M-MLV Reverse Transcriptase (M1705, Promega, Madison, USA) and qPCR was conducted utilizing SYBR green kit (Promega, Madison, Wisconsin, USA) and a CFX96 PCR machine (Bio-Rad, Hercules, USA). Gene expression was normalized to 62-*microglobulin* and expressed relative to the saline group. Primer sequences are displayed in Table S1.

### Plasma measurements

Plasma TG and TC levels were measured using Cobas Triglycerides (106571) or Cobas Total Cholesterol (106570) enzymatic reagenets (Roche Diagnostics, Mannheim, Germany). 200 µL reagent (reagent was undiluted for TG and 3x diluted for TC) was added to 7.5 µL plasma sample (5x diluted) and incubated at room temperature for 30 minutes prior to measuring transmittance at 492 nm versus 650 nm (for TG) or at 505 nm versus 650 nm (for TC). To calculate total TC exposure, the area under the curve was determined from regularly monitored plasma TC levels over time. For quantification of high-density lipoprotein cholesterol (HDL-C), apolipoprotein B (ApoB) containing lipoproteins were precipitated from plasma by adding 20% polyethylene glycol in 200 mM glycine-buffered saline (pH 10), and TC was measured in the supernatant as described above. Non-HDL-C plasma levels were calculated by subtracting plasma HDL-C from plasma TC. Plasma alanine aminotransferase (ALT) activity was determined by the Alanine Aminotransferase Activity Assay Kit (Sigma-Aldrich, St. Louis, USA) according to the manufacturer's protocol.

### Liver histology, lipid, and water content

Formaldehyde-fixated, paraffin-embedded liver was cross-sectioned into 5 µm sections. A section was stained with hematoxylin and eosin, and another section was used to quantify monocyte chemoattractant protein-1 (MCP-1) content by the use of rabbit polyclonal anti-mouse MCP-1 antibody (1:100; ab25124; Abcam, Cambridge, UK) and secondary goat anti-rabbit IgG (1:200; P0448; Dako, Heverlee, The Netherlands). Lipids were extracted from frozen liver tissue using a modified version of the Bligh and Dyer protocol [16], and TG and TC levels were measured as described above. To determine water content of the liver, 50-150 mg tissue was weighed prior to and after freeze-drying for 5 days (Alpha 1-4 LSCbasic, Martin Christ, Osterode am Harz, Germany).

### In vivo organ uptake of TRL-mimicking particles

TRL-mimicking particles labeled with glycerol tri[³H]oleate and [¹⁴C]cholesteryl oleate were prepared as described before [17]. Particles were injected into the tail vein (1 mg TG per mouse in 200 µL saline) 15 minutes before the mice were killed via CO₂ asphyxiation. Subsequently, mice were perfused transcardially with ice-cold PBS for 5 minutes before various (parts of) organs (approximately 50-150 mg) were collected and dissolved in 0.5 mL Solvable (6NE9100, PerkinElmer, Waltham, USA) at 56°C overnight, after which 5.0 mL Ultima Gold (6013329, PerkinElmer, Waltham, USA) was added. ³H-activity and ¹⁴C-activity were measured simultaneously with a scintillation counter (Tri-Carb 2910 TR, PerkinElmer, Waltham, USA) and expressed as a percentage of the injected dose per whole tissue. Lipolysis index was calculated as the ratio between ³H-activity and ¹⁴C-activity per tissue.

### Hepatic VLDL-TG and VLDL-ApoB production assay

Mice were anesthetized by intraperitoneal injection with a combination of ventranquil (6.25 mg/kg), midazolam (6.25 mg/kg), and fentanyl (0.31 mg/kg) in a total volume of 8 µL/g body weight, followed by subcutaneous injections of 50 µL every 45 minutes to sustain anesthesia. To label newly synthesized ApoB, mice were then injected intravenously with 10 µCi Tran[³⁵S] label (IS-103; Hartmann Analytic, Braunschweig, Germany). After 30 minutes, mice were injected intravenously with 10% Triton WR-1339 (5 µL/g body weight, Sigma-Aldrich Saint Louis, USA) to block LPL activity and thus VLDL-TG clearance. Blood was collected via the tail vein prior to and 15, 30, 60, and 90 minutes after the Triton WR-1339 injection for quantification of plasma TG levels as described above. At 120 minutes, mice were exsanguinated via the eye. Plasma was ultracentrifuged in a density gradient, after which VLDL was isolated by aspiration [18]. ³⁵S-activity was measured in the acquired VLDL with and without the addition of 400 µL isopropanol to precipitate ApoB, and ³⁵S-activity was expressed as disintegrations per minute (DPM) per mL, and VLDL-TG and -TC was measured as described above. A colorimetric assay was used to determine phospholipids (PL) (INstruchemie, Delfzijl, The Netherlands) and protein content (Thermo Fisher Scientific, Waltham, USA) of the particles.

### Atherosclerosis quantification

Formaldehyde-fixated, paraffin-embedded hearts were cross-sectioned (5 µm) perpendicular to the axis of the aorta throughout the aortic root area. Sections were stained with hematoxylin-phloxine-saffron for histological analysis of lesion area as described previously [13]. Four subsequent sections (50 µm apart) were analyzed per valve starting from the first appearance of open aortic valve leaflets. Lesions were categorized by severity (mild: types I-III or severe: types IV-V) according to the guidelines of the American Heart Association adapted for mice [19]. Quantification of macrophage content was performed using rat monoclonal anti-mouse MAC-3 antibody (1:1000; 550292; BD Pharmingen, San Diego, USA) and secondary goat anti-rat IgG (MP7444; Vector Laboratories Inc., Burlingame, USA). Monoclonal mouse antibody (1:1000; M0851; Dako, Heverlee, The Netherlands) against smooth muscle cell actin and secondary goat anti-mouse IgG (1:400; K4003; Dako, Heverlee, The Netherlands) were used to quantify smooth muscle cells. The immunoperoxidase complexes on the secondary antibodies were visualized with Nova Red (SK-4800, Vector Laboratories Inc., Burlingame, USA) and Liquid Dab + Substrate Chromogen System (K3468, Dako, Heverlee, The Netherlands) for the macrophage and smooth muscle cell quantification, respectively. A solution of direct red and fast green (both 1:1000; 365548-5G and F7258S, respectively; Sigma Aldrich, St. Louis, USA) was used to stain collagen. Total lesion area and composition were determined using Image J software (version 1.52a, National Institutes of Health, Bethesda, Maryland). Lesion stability index was calculated per mouse by dividing the sum of relative collagen- and smooth muscle cell-positive lesion area by relative macrophage-positive area.

### Toxicology study in cynomolgus monkeys

The safety of a liver-targeted ASO designed to inhibit human *ANGPTL4* mRNA was assessed in cynomolgus monkeys at Charles River Laboratories (Tranent, UK). The monkeys were group-housed, had *ad libitum* access to water, and were fed with a balanced diet (Special Diet Services Mazuri Primate Diet) enriched with fruits, vegetables, nuts and seeds. For a period of four weeks, the animals received weekly subcutaneously injections with saline (n=5 males and n=5 females), 3 mg/kg anti*-ANGPTL4* ASO (n=3 males and n=3 females), 10 mg/kg anti*-ANGPTL4* ASO (n=3 males and n=3 females), or 30 mg/kg anti-*ANGPTL4* ASO (n=5 males and n=5 females). After the treatment period, three males and three females per group were euthanized for toxicological evaluation. Another two males and two females of the monkeys treated with saline or 30 mg/kg Anti-*ANGPTL4* ASO were euthanized after a four week washout period. The animal procedures were performed in accordance with the OECD Principles of Good Laboratory Practice as incorporated into the United Kingdom Statutory Instrument for GLP.

### Statistical analysis

Data was tested for normality, and in case of normal distributions, statistical analyses between groups were performed by one-way ANOVA with post-hoc Šídák's test. In the VLDL production experiment, one-way ANOVA with post-hoc Šídák's test was performed on calculated slopes of TG. In case of non-normal distributions, Kruskal-Wallis tests with a post-hoc Dunn's test were used instead of one-way ANOVA. Two-way ANOVA or mixed-effects models with post-hoc Tukey's test were used for repeated measurement in case of sequential sampling and for the fasting-feeding measurements. When data of repeated measurements was not normally distributed, two-way ANOVA tests were performed on ranked values. Additional two-way ANOVA was used in which the saline group was excluded to test for main and interaction effects of anti-Angptl3 and anti-Angptl4 ASO treatments. Pearson correlation was used to calculate correlation coefficients. Statistical outliers were removed using Grubb's test. Statistical analyses were performed with GraphPad Prism software, version 9.3.1 (GraphPad, La Jolla, California). P<0.05 was considered statistically significant. Data are presented as means ± SEM.

### Liver-targeted Angptl3 and Angptl4 silencing strongly lowers plasma lipid levels

To compare the effects of liver-targeted Angptl3 and Angptl4 silencing alone and in combination on hyperlipidemia, female APOE*3-Leiden.CETP mice were treated with saline, negative (scrambled) ASO, anti-Angptl3 ASO, anti- Angptl4 ASO, or anti-Angptl3 ASO + anti-Angptl4 ASO for a total duration of two weeks. Suppression of hepatic Angptl3 and Angptl4 expression was confirmed (Angptl3 downregulation of 71% and 64% by Angptl3 and combined Angptl3/4 silencing, respectively, and Angptl4 downregulation of 73% and 82% by Angptl4 and combined Angptl3/4 silencing, respectively) (Fig. 1A). While body weight (Fig. 1B), lean mass (Fig. 1C), and fat mass (Fig. 1D) were unchanged, combined Angptl3/4 silencing reduced gonadal WAT (gWAT) and subcutaneous WAT (sWAT) weights (Fig. 1E) without affecting food intake (Fig. 1F). Both Angptl3 and Angptl4 silencing strongly lowered plasma TG (Fig. 1G), TC (Fig. 1H), and non-HDL-C levels (Fig. 1I) compared with negative ASO treatment. The reductions in plasma TG, TC, and non-HDL-C levels by combined Angptl3/4 silencing were comparable to those induced by the single ASO treatments. In contrast, only combined Angptl3/4 silencing increased plasma HDL-C levels compared with negative ASO treatment (Fig. 1J).

### Liver-targeted Angptl4 silencing increases lipid uptake by brown adipose tissue and reduces hepatic VLDL production

Next, we investigated if liver-targeted silencing of Angptl3 and Angptl4 reduced plasma lipid levels by similar or different mechanisms. Firstly, 4-hour-fasted mice were injected with TRL-mimicking particles that were double-labeled with glycerol tri[3H]oleate and [14C]cholesteryl oleate, reflecting TG-derived FA and TRL-remnant uptake, respectively. Angptl4 silencing and combined Angptl3/4 silencing elevated [3H]oleate uptake by interscapular brown adipose tissue (iBAT) and liver, while hepatic [14C]cholesteryl oleate uptake was unaffected, thereby increasing the 3H/14C uptake ratio in the liver (Fig. 2A-C). In contrast, Angptl3 silencing did not increase [3H]oleate uptake by BAT, but elevated hepatic [3H]oleate and [14C]cholesteryl oleate uptake (Fig. 2A and 2B).

Secondly, we evaluated the VLDL-TG production rate as well as VLDL-ApoB production in vivo. Interestingly, both Angptl3 and Angptl4 silencing reduced hepatic TG production with a corresponding reduction in VLDL-[35S]ApoB production (Fig. 2D and 2E). These reductions were stronger with Angptl4 and combined Angptl3/4 silencing compared with Angptl3 silencing, and accompanied by significant reductions in hepatic expression of Apob and microsomal triglyceride transfer protein (Mttp), genes involved in VLDL assembly (Fig. 2F). Likewise, all treatments decreased TG, TC, PL, and protein content within the VLDL fraction, while these reductions were the largest and only significant with Angptl4 and combined Angptl3/4 silencing (Fig. 2G-J).

### Liver-targeted Angptl3 and Angptl4 silencing does not induce steatosis or inflammation

Unexpectedly, Angptl3, Angptl4, and combined Angptl3/4 silencing increased liver weight (Fig. 6A), without increasing relative TG (Fig. 6B), TC (Fig. 6C), protein (Fig. 6D), or water content (Fig. 6E) compared with negative ASO treatment. Hepatocytes were enlarged with coinciding vacuolization of the cytoplasm (Fig. 6F). Notably, livers of the negative ASO groups were unaffected. ASO-related toxicity is common in mice and often manifests in liver injury and inflammation [20]. However, hallmarks of ASO toxicity [20, 21] including the presence of inflammatory infiltrates, formation of basophilic granules upon ASO accumulation, signs of apoptosis or necrosis, and expression of genes encoding pro-inflammatory factors were not observed (Fig. 6F-I). Furthermore, whereas ASO-related hepatotoxicity can elevate plasma ALT activity 50- to 200-fold to up to 1000 U/L in mice [21], anti-Angptl4 and combined ASO treatment only non-significantly induced a 3-fold elevation in plasma ALT activity up to 60 U/L (Fig. 6G), indicating no severe liver damage. Of note, all ASO treatments increased hepatic expression of x-box binding protein 1 (Xbp1), but not sortilin 1 (Sort1), which are markers of endoplasmic reticulum (ER) stress (Fig. 6J). Collectively, these data indicate an ER stress response to some extent, without development of hepatic steatosis or inflammation upon Angptl3 or Angptl4 silencing. Importantly, a 4-week toxicology study in cynomolgus monkeys showed that anti-ANGPTL4 ASO treatment (weekly subcutaneous injections of up to 30 mg/kg) was well tolerated and did not induce any adverse effects, such as increased liver weight, abnormal hepatic microscopic findings, or elevated plasma levels of ALT and aspartate transaminase (AST) (Tables 1-3).

**Table 1. Liver weight of cynomolgus monkeys**

| | **Males** | | | | **Females** | | | |
|---|---|---|---|---|---|---|---|---|
| **Group** | **1** | **2** | **3** | **4** | **1** | **2** | **3** | **4** |
| **Dose (mg/kg/dose)** | **0** | **3** | **10** | **30** | **0** | **3** | **10** | **30** |
| **N size** | **3** | **3** | **3** | **3** | **3** | **3** | **3** | **3** |
| Liver weight (including gallbladder) (g) | 68.8±6.7 | 61.5±1.1 | 67.2±5.6 | 80.4±4.5 | 66.0±13.2 | 59.2±3.8 | 56.8±9.6 | 55.1±5.3 |

**Table 2. Hepatic microscopic findings in cynomolgus monkeys**

| | **Mates** | | | | **Females** | | | |
|---|---|---|---|---|---|---|---|---|
| **Group** | **1** | **2** | **3** | **4** | **1** | **2** | **3** | **4** |
| **Dose (mg/kg/dose)** | **0** | **3** | **10** | **30** | **0** | **3** | **10** | **30** |
| **No. animals examined** | **3** | **3** | **3** | **3** | **3** | **3** | **3** | **3** |
| **Livers (No. examined)** | (3) | (3) | (3) | (3) | (3) | (3) | (3) | (3) |
| Basophilic granules, hepatocellular (all of minimal severity) | 0 | 0 | 1 | 1 | 0 | 0 | 1 | 3 |
| Basophilic granules, Kuppfer cell, (of minimal severity) | 0 | 0 | 0 | 0 | 0 | 0 | 0 | 1 |

**Table 3. Liver toxicity markers in cynomolgus monkeys. AST, aspartate transaminase. ALT, alanine transaminase. *p<0.05, according to one-way ANOVA and following Dunn's multiple-comparison test.**

| | **Males** | | | | **Females** | | | |
|---|---|---|---|---|---|---|---|---|
| **Group** | **1** | **2** | **3** | **4** | **1** | **2** | **3** | **4** |
| **Dose (mg/kg/dose)** | **0** | **3** | **10** | **30** | **0** | **3** | **10** | **30** |
| **N size** | **5** | **3** | **3** | **5** | **5** | **3** | **3** | **5** |
| **Day -12** | | | | | | | | |
| AST (U/L) | 40.0±5.8 | 64.0±24.3 | 44.3±10.1 | 37.4±6.5 | 36.4±6.9 | 36.0±6.0 | 55.7±11.0* | 48.6±7.4 |
| ALT (U/L) | 46.4±10.5 | 90.3±64.7 | 44.0±4.6 | 50.4±6.6 | 45.8±10.4 | 41.0±2.0 | 47.0±13.5 | 52.4±9.6 |
| **Day 25** | | | | | | | | |
| AST (U/L) | 34.0±3.9 | 37.7±4.6 | 25.0±5.6 | 29.8±5.5 | 30.6±3.2 | 25.0±4.6 | 28.0±1.7 | 32.2±10.8 |
| ALT (U/L) | 42.6±11.1 | 56.0±18.7 | 39.0±3.6 | 65.4±20.4 | 42.4±10.0 | 44.0±10.0 | 40.7±5.5 | 52.0±16.9 |

### Liver-targeted Angptl3 and Angptl4 silencing lowers plasma TG in refed and fasted state, respectively

Since short-term liver-targeted Angptl3 and Angptl4 silencing potently attenuated hyperlipidemia, we next assessed to what extent these treatments would also reduce atherosclerosis development over a treatment period of 12 weeks. To minimize hepatic enlargement, we first tested various lower ASO concentrations for their potency to downregulate hepatic Angptl3 and Angptl4 expression (data not shown). We accordingly reduced the dosage from 1.25 mg/kg to 0.30 mg/kg, with the exception of the negative ASO (0.60 mg/kg) to match the total amount of ASO administered to the combined group (0.30 mg/kg of both anti-Angptl3 and anti-Angptl4 ASO). Suppression of hepatic Angptl3 and Angptl4 expression by the ASO treatments at this dose was confirmed (Fig. 3A and 3B). Angptl3 silencing and combined Angptl3/4 silencing downregulated Angptl3 expression by 58% and 56% in the fasted state, and by 35% and 38% in the refed state, respectively (Fig. 3A). Angptl4 silencing and combined Angptl3/4 silencing downregulated Angptl4 expression by 75% and 75% in the fasted state, and by 80% and 81% in the refed state, respectively (Fig. 3B). With this lower dosage, anti-Angptl4 and combined ASO treatment, but not anti-Angptl3 ASO treatment, still increased liver weight (Fig. 3C) and again only mildly elevated plasma ALT activity (Fig. 7A), without increasing relative hepatic TG or TC content (Fig. 7B and 7C). In line with the short-term study, Angptl3 or Angptl4 silencing did not alter body weight (Fig. 3D) or lean mass (Fig. 3E), while Angptl4 and combined Angptl3/4 silencing decreased fat mass after eight weeks (Fig. 3F), and lowered the weights of gWAT and sWAT depots isolated after 12 weeks (Fig. 3G) compared with negative ASO-treated mice. These effects were independent of total food intake, since Angptl4 silencing tended to increase food intake from week ten onwards and combined Angptl3/4 silencing significantly increased food intake from week four onwards (Fig. 3H and 7D).

We confirmed that Angptl3 and Angptl4 silencing lowered plasma TC and non-HDL-C levels, and observed that Angptl4 silencing caused greater reductions than Angptl3 silencing from four weeks onwards (Fig. 4A and 4B). In addition, plasma TG levels were only significantly lowered with Angptl4 and combined Angptl3/4 silencing (Fig. 4C). Plasma HDL-C levels were elevated by combined Angptl3/4 silencing throughout the study and by Angptl4 silencing in week eight when compared with negative ASO treatment (Fig. 4D). Since endogenous ANGPTL3 and ANGPTL4 activity is highly dependent on nutritional status, plasma lipid levels were determined after 16 hours fasting or after 12 hours fasting followed by 4 hours refeeding, at week 12. Accordingly, Angptl3 silencing lowered plasma TG levels specifically after refeeding (Fig. 4E; anti-Angptl3 ASO effect by two-way ANOVA: P<0.001), while Angptl4 silencing lowered TG levels in the fasted state only (Fig. 4E; anti-Angptl4 ASO effect by two-way ANOVA: P=0.007). Combined Angptl3/4 silencing reduced plasma TG levels in both nutritional states, albeit non-significantly in the post-hoc analysis (Fig. 4E). In contrast to plasma TG levels, TC levels were consistently reduced by all ASO treatments in the fasted as well as the refed state (Fig. 4F; anti-Angptl3 ASO and anti-Angptl4 ASO effects by two-way ANOVA: P<0.001). These results suggest that the TG-lowering effects of liver-targeting Angptl3 and Angptl4 silencing are dependent on nutritional state, while combined treatment consistently reduces plasma TG levels along with TC levels in various physiological settings.

### Liver-targeted Angptl3 and Angptl4 silencing attenuates atherosclerosis development

In line with reduced plasma non-HDL-C levels, all treatments considerably reduced atherosclerotic lesion area throughout the aortic root (Fig. 5A and 5B). In addition, all treatments decreased lesion severity, evident from a decline in the relative amount of severe lesions and an increase in the relative amount of mild lesions compared with the negative ASO group (Fig. 5C). Angptl4 silencing induced a greater attenuation of atherosclerosis development than Angptl3 silencing , while the effects of Angptl4 and combined Angptl3/4 silencing were comparable. These effects related to cumulative TC and TG exposure throughout the study (Fig. 5D and 5E), which correlated strongly with the average lesion area (Fig. 5F and 5G), suggesting that the alleviation of atherosclerosis development by Angptl3 and Angptl4 silencing was driven by the reduction in hypercholesteremia as well as hypertriglyceridemia. Both Angptl4 and combined Angptl3/4 silencing, but not Angptl3 silencing, strongly lowered the relative macrophage content of the lesions (Fig. 5H; anti-Angptl4 ASO effect by two-way ANOVA: P<0.001) and tended to increase smooth muscle cell and collagen content (Fig. 5H; anti-Angptl4 ASO effect by two-way ANOVA: P=0.028 and P=0.029 for smooth muscle cell and collagen content, respectively). Together, these characteristics contributed to an increased lesion stability index after Angptl4 and combined Angptl3/4 silencing (Fig. 5I), which tended to be even further elevated after combined Angptl3/4 silencing compared with Angptl4 silencing (Fig. 5I; interaction effect by two-way ANOVA: p=0.079). Representative pictures of all histological stainings of the atherosclerotic lesions are shown (Fig. 5J).

### References

1. Dijk, W. and S. Kersten, Regulation of lipoprotein lipase by Angptl4. Trends in Endocrinology & Metabolism, 2014. 25(3): p. 146-155.
2. Kersten, S., et al., Caloric restriction and exercise increase plasma ANGPTL4 levels in humans via elevated free fatty acids. Arteriosclerosis, thrombosis, and vascular biology, 2009. 29(6): p. 969-974.
3. Dijk, W., et al., ANGPTL4 mediates shuttling of lipid fuel to brown adipose tissue during sustained cold exposure, elife, 2015. 4: p. e08428.
4. van Eenige, R., et al., Angiopoietin-like 4 governs diurnal lipoprotein lipase activity in brown adipose tissue. Mol Metab, 2022. 60: p. 101497.
5. Romeo, S., et al., Rare loss-of-function mutations in ANGPTL family members contribute to plasma triglyceride levels in humans. The Journal of clinical investigation, 2009. 119(1): p. 70-79.
6. Kersten, S., New insights into angiopoietin-like proteins in lipid metabolism and cardiovascular disease risk. Current opinion in lipidology, 2019. 30(3): p. 205-211.
7. Gusarova, V., et al., ANGPTL3 blockade with a human monoclonal antibody reduces plasma lipids in dyslipidemic mice and monkeys. Journal of lipid research, 2015. 56(7): p. 1308-1317.
8. Graham, M.J., et al., Cardiovascular and metabolic effects of ANGPTL3 antisense oligonucleotides. N Engl J Med, 2017. 377: p. 222-232.
9. Dewey, F.E., et al., Genetic and Pharmacologic Inactivation of ANGPTL3 and Cardiovascular Disease. N Engl J Med, 2017. 377(3): p. 211-221.
10. Raal, F.J., et al., Evinacumab for Homozygous Familial Hypercholesterolemia. N Engl J Med, 2020. 383(8): p. 711-720.
11. Reeskamp, L.F., et al., ANGPTL3 Inhibition With Evinacumab Results in Faster Clearance of IDL and LDL apoB in Patients With Homozygous Familial Hypercholesterolemia-Brief Report. Arterioscler Thromb Vasc Biol, 2021. 41(5): p. 1753-1759.
12. Westerterp, M., et al., Cholesteryl ester transfer protein decreases high-density lipoprotein and severely aggravates atherosclerosis in APOE*3-Leiden mice. Arterioscler Thromb Vase Biol, 2006. 26(11): p. 2552-9.
13. Gijbels, M.J., et al., Progression and regression of atherosclerosis in APOE3-Leiden transgenic mice: an immunohistochemical study. Atherosclerosis, 1999. 143(1): p. 15-25.
14. Zadelaar, S., et al., Mouse models for atherosclerosis and pharmaceutical modifiers. Arterioscler Thromb Vase Biol, 2007. 27(8): p. 1706-21.
15. van den Hoek, A.M., et al., APOE*3-Leiden.CETP transgenic mice as model for pharmaceutical treatment of the metabolic syndrome. Diabetes Obes Metab, 2014. 16(6): p. 537-44.
16. Bligh, E.G. and W.J. Dyer, A rapid method of total lipid extraction and purification. Can J Biochem Physiol, 1959. 37(8): p. 911-7.
17. Ying, Z., et al., A simplified procedure to trace triglyceride-rich lipoprotein metabolism in vivo. Physiological Reports, 2021. 9(8): p. e14820.
18. Redgrave, T.G., D.C. Roberts, and C.E. West, Separation of plasma lipoproteins by density-gradient ultracentrifugation. Anal Biochem, 1975. 65(1-2): p. 42-9.
19. Zadelaar, A.S.M., et al., Dual PPARα/γ agonist tesaglitazar reduces atherosclerosis in insulin-resistant and hypercholesterolemic ApoE* 3Leiden mice. Arteriosclerosis, thrombosis, and vascular biology, 2006. 26(11): p. 2560-2566.
20. Frazier, K.S., Antisense oligonucleotide therapies: the promise and the challenges from a toxicologic pathologist's perspective. Toxicol Pathol, 2015. 43(1): p. 78-89.
21. Swayze, E.E., et al., Antisense oligonucleotides containing locked nucleic acid improve potency but cause significant hepatotoxicity in animals. Nucleic Acids Res, 2007. 35(2): p. 687-700.

### Items

1. An ANGPTL4 inhibitor comprising an antisense oligonucleotide consisting of 10-50 nucleotides in length, wherein 10-30 consecutive nucleotides are complementary to SEQ ID NO.1 or SEQ ID NO. 2, and has at least one affinity-enhancing nucleotide analogue and comprises at least one phosphorothioate internucleoside linkage, and wherein the ANGPTL4 inhibitor is for use in treatment, alleviation, pre-emptive treatment, prevention or prophylaxis of a cardiovascular disease in a mammal.
2. The ANGPTL4 inhibitor according to item 1, wherein the mammal is a human.
3. The ANGPTL4 inhibitor according to item 1 or 2, wherein the cardiovascular disease is atherosclerosis.
4. The ANGPTL4 inhibitor according to any of the preceding items, wherein the use is for lowering of plasma triglycerides and/or total cholesterol, such as for treatment of high levels of plasma triglycerides and/or high levels of total cholesterol.
5. The ANGPTL4 inhibitor according to any of the preceding items, wherein the use is for reducing circulating triglyceride-rich lipoproteins, such as for treatment of high levels of circulating triglyceride-rich lipoproteins.
6. The ANGPTL4 inhibitor according to any of the preceding items, wherein the use is for increasing plasma HDL-C levels, such as for treatment of low plasma HDL-C levels.
7. The ANGPTL4 inhibitor according to any of the preceding items, wherein the use is for reducing VLDL-ApoB production, such as for treatment of high VLDL-ApoB levels.
8. The ANGPTL4 inhibitor according to any of the preceding items, wherein the use is for prevention of atherosclerotic lesions.
9. The ANGPTL4 inhibitor according to any of the preceding items, wherein the use is for reducing atherosclerotic lesion size.
10. The ANGPTL4 inhibitor according to any of the preceding items, wherein the use is for reducing the number of severe atherosclerotic lesions.
11. The ANGPTL4 inhibitor according to any of the preceding items, wherein the use is for improving the atherosclerotic lesion index in a patient.
12. The ANGPTL4 inhibitor according to any of the preceding items, wherein the inhibitor inhibits the expression of ANGPTL4.
13. The ANGPTL4 inhibitor according to any of the preceding items, wherein the inhibitor comprises an antisense oligonucleotide which is complementary to at least 10, such as at least 11, 12, 13, 14, 15, 16, 17, 18, 19 or 20 consecutive nucleotides of any one of position 1732 to 1759, from position 234-261, from position 1264-1296 of SEQ ID NO. 1 and/or from position 2800-2872, from position 3415-3442, from position 4968-4994 of SEQ ID NO.2 or a combination thereof.
14. The ANGPTL4 inhibitor according to item 13, wherein the inhibitor comprises an antisense oligonucleotide which is complementary to at least 10, such as at least 11, 12, 13, 14, 15, 16, 17, 18, 19 or 20 consecutive nucleotides of 1264-1295, such as is fully complementary to nucleotides 1269-1295 of SEQ ID NO. 1.
15. The ANGPTL4 inhibitor according to any of the preceding items, wherein the modified nucleotide is selected from the group consisting of a bridged nucleic acid such as LNA, cET, ENA, BNA, UNA, 2'Fluoro modified nucleotide, 2'0-Methyl modified nucleotide, 2'-Methoxyethyl modified nucleotide and a combination thereof.
16. The ANGPTL4 inhibitor according to any of the preceding items, wherein the inhibitor comprises a gapmer antisense oligonucleotide having a gap of at least 5 consecutive DNA nucleotides
17. The ANGPTL4 inhibitor according to any of the preceding items, comprising a sequence selected from the group consisting of SEQ ID N0.47, SEQ ID NO.159, SEQ ID NO.161, SEQ ID NO.163, SEQ ID NO.165, SEQ ID NO.173, SEQ ID NO.179, SEQ ID NO.186, SEQ ID NO.187, SEQ ID NO.3, SEQ ID NO.4, SEQ ID NO.5, SEQ ID NO.6, SEQ ID NO.7, SEQ ID NO.8, SEQ ID NO.9, SEQ ID NO.10, SEQ ID NO.11, SEQ ID NO.12, SEQ ID NO.13, SEQ ID NO.14, SEQ ID NO.15, SEQ ID NO.16, SEQ ID NO.17, SEQ ID NO.18, SEQ ID NO.19, SEQ ID NO.20, SEQ ID NO.21, SEQ ID NO.22, SEQ ID NO.23, SEQ ID NO.24, SEQ ID NO.25, SEQ ID NO.26, SEQ ID NO.27, SEQ ID NO.28, SEQ ID NO.29, SEQ ID NO.30, SEQ ID NO.31, SEQ ID NO.32, SEQ ID NO.33, SEQ ID NO.34, SEQ ID NO.35, SEQ ID NO.36, SEQ ID NO.37, SEQ ID NO.38, SEQ ID NO.39, SEQ ID NO.40, SEQ ID NO.41, SEQ ID NO.42, SEQ ID NO.43, SEQ ID NO.44, SEQ ID NO.45, SEQ ID NO.46, SEQ ID NO.47, SEQ ID NO.48, SEQ ID NO.49, SEQ ID NO.50, SEQ ID NO.51, SEQ ID NO.52, SEQ ID NO.53, SEQ ID NO.54, SEQ ID NO.55, SEQ ID NO.56, and or a combination of any of those.
18. The ANGPTL4 inhibitor according to any of the preceding items, wherein the antisense oligonucleotide is selected from the group consisting of +C*+A*+A*+T*G*T*G*A*C*T*G*A*G*T*+C*+C*+G (SEQIDNO.47), +C*+A*+A*T*G*T*G*A*C*T*G*A*G*T*+C*+C*+G (SEQIDNO.47), +C*+A*+A*T*G*T*G*A*C*T*G*A*G*+T*+C*+C*+G (SEQIDNO.47), +T*+C*+G*A*G*A*T*G*A*A*C*G*G*+A*+G*+A (SEQIDNO.159), +A*+A*+C*T*T*A*G*A*G*A*A*C*C*G*+C*+G*+A (SEQIDNO.161), +T*+T*+A*G*A*G*A*A*C*C*G*C*G*+A*+G*+T (SEQIDNO.163), +T*+C*+G*A*A*A*T*G*A*G*T*C*T*G*+C*+A*+C (SEQIDNO.165), +C*+T*+T*A*A*C*A*G*T*G*G*A*T*G*+A*+C*+C (SEQIDNO.173), +T*+A*+G*C*A*C*G*G*C*G*G*T*G*+G*+C*+G (SEQIDNO.179), +A*+G*+T*A*C*T*G*G*C*C*G*T*T*G*+A*+G*+G (SEQIDNO.186), +G*+A*+A*G*T*A*C*T*G*G*C*C*G*T*+T*+G*+A (SEQIDNO.187), +A*G*G*C*T*A*A*G*T*T*A*G*T*G*G*+C*+C (SEQIDNO.3), +G*+C*C*A*G*A*T*A*G*G*C*T*A*A*+G*+T*+T (SEQIDNO.4), +C*+T*G*+G*A*A*A*G*A*A*T*C*G*G*+A*+T*+C (SEQIDNO.5), +C*G*+C*T*G*G*A*A*A*G*A*A*T*+C*+G*+G*+A (SEQIDNO.6), +G*C*+T*A*G*T*C*T*C*G*A*C*A*G*C*+A*+G (SEQIDNO.7), +G*+A*T*C*C*T*C*A*T*G*A*T*A*G*G*+C*+C (SEQIDNO.8), +G*+G*C*C*G*T*C*G*G*A*G*C*A*C*C*G*+C (SEQIDNO.9) +C*+A*+T*G*C*T*G*T*G*G*T*T*C*G*+A*+G*+A (SEQIDNO.10), +C*+G*T*G*C*G*C*C*A*G*G*A*C*A*+T*+T*+C (SEQIDNO.11), +C^{∗}+G^{∗}+T^{∗}G^{∗}C^{∗}G^{∗}C^{∗}C^{∗}A^{∗}G^{∗}G^{∗}A^{∗}C^{∗}+A^{∗}+T^{∗}+T (SEQIDNO.12), +T*C*C*G*T*G*C*G*C*C*A*G*G*A*+C*A*+T (SEQIDNO.13), +G^{∗}G^{∗}A^{∗}G^{∗}T^{∗}C^{∗}C^{∗}G^{∗}T^{∗}G^{∗}C^{∗}G^{∗}C^{∗}C^{∗}+A^{∗}G^{∗}+G (SEQIDNO.14), +T*+G*C*G*C*T*C*C*G*C*G*T*G*T*T*+C*+G (SEQIDNO15) +T*+G*C*G*C*T*C*C*G*C*G*T*G*T*+T*+C (SEQIDNO.16), +G*+T*G*C*G*C*T*C*C*G*C*G*T*G*T*+T (SEQIDNO.17), +G*+T*+C*G*A*A*A*T*G*A*G*T*C*T*+G*+C*+A (SEQIDNO.18), +G^{∗}+C^{∗}+A^{∗}C^{∗}G^{∗}T^{∗}C^{∗}T^{∗}G^{∗}G^{∗}A^{∗}G^{∗}G^{∗}C^{∗}+T^{∗}+C^{∗}+A (SEQIDNO.19), +T*+T*+G*A*G*C*A*C*G*T*C*T*G*G*+A*+G*+G (SEQIDNO.20), +G*+G*+A*T*C*G*C*A*G*T*G*C*C*G*+C*+A*+A (SEQIDNO.21), +G*C*+T*G*A*A*T*T*C*G*C*A*G*G*+T*G*+C (SEQIDNO.23), +G*+A*+T*C*G*C*A*G*T*G*C*C*G*+C*+A*+A (SEQIDNO.24), +G*+T*+G*T*T*G*T*A*A*C*C*T*C*T*+T*+G*+T (SFQIDNO25) +C^{∗}+C^{∗}+G^{∗}T^{∗}G^{∗}T^{∗}T^{∗}G^{∗}T^{∗}A^{∗}A^{∗}C^{∗}C^{∗}T^{∗}+C^{∗}+T^{∗}+T (SEQIDNO.26), +C^{∗}+C^{∗}+T^{∗}+C^{∗}A^{∗}T^{∗}G^{∗}G^{∗}T^{∗}C^{∗}T^{∗}A^{∗}G^{∗}G^{∗}+T^{∗}+G^{∗}+C (SEQIDNO.27), +C^{∗}A^{∗}+C^{∗}C^{∗}T^{∗}C^{∗}A^{∗}T^{∗}G^{∗}G^{∗}T^{∗}C^{∗}T^{∗}A^{∗}G^{∗}+G^{∗}+T (SEQIDNO.28), +G^{∗}G^{∗}C^{∗}G^{∗}C^{∗}C^{∗}T^{∗}C^{∗}T^{∗}G^{∗}A^{∗}A^{∗}T^{∗}T^{∗}+A^{∗}+C^{∗}+T (SEQIDNO.29), +C*+G*+T*G*G*C*G*C*C*T*C*T*G*A*+A*+T*+T (SEQIDNO.30), +T*C*G*T*G*G*C*G*C*C*T*C*T*G*A*+A*+T (SEQIDNO.31), +C*+C*+G*C*C*T*T*G*T*A*G*G*C*T*T*C*+C (SEQIDNO.32), +A*+C*+T*C*G*G*C*G*T*T*G*C*C*A*T*C*+C (SEQIDNO.33), +G*+C*C*G*T*G*T*C*C*T*C*G*C*C*A*C*+C (SEQIDNO.34), +G*+A*G*G*T*C*G*T*G*A*T*C*C*T*G*+G*+T (SEQIDNO.35), +G^{∗}+C^{∗}+G^{∗}G^{∗}A^{∗}G^{∗}G^{∗}T^{∗}C^{∗}G^{∗}T^{∗}G^{∗}A^{∗}T^{∗}C^{∗}+C^{∗}+T (SEQIDNO.36), +C^{∗}C^{∗}T^{∗}G^{∗}C^{∗}G^{∗}G^{∗}A^{∗}G^{∗}G^{∗}T^{∗}C^{∗}G^{∗}T^{∗}+G^{∗}+A^{∗}+T (SEQIDNO.37), +C^{∗}+T^{∗}+C^{∗}T^{∗}T^{∗}G^{∗}G^{∗}C^{∗}G^{∗}C^{∗}A^{∗}G^{∗}T^{∗}T^{∗}+C^{∗}+T^{∗}+T (SEQIDNO.38), +G^{∗}+C^{∗}+T^{∗}C^{∗}T^{∗}T^{∗}G^{∗}G^{∗}C^{∗}G^{∗}C^{∗}A^{∗}G^{∗}T^{∗}+T^{∗}+C^{∗}+T (SEQIDNO.39), +G*+G*+C*T*C*T*T*G*G*C*G*C*A*G*T*+T*+C (SEQIDNO.40), +G^{∗}+G^{∗}T^{∗}G^{∗}C^{∗}C^{∗}A^{∗}A^{∗}A^{∗}C^{∗}C^{∗}A^{∗}C^{∗}C^{∗}+A^{∗}+G^{∗}+C (SEQIDNO.41), +G*+G*+A*+G*C*G*G*A*A*G*T*A*C*T*G*+G*+C (SEQIDNO.42), +A*+T*+G*G*A*G*C*G*G*A*A*G*T*+A*+C*+T*+G (SEQIDNO.43), +G*+G*+A*T*G*G*A*G*C*G*G*A*A*G*+T*+A*+C (SEQIDNO.44), +G^{∗}+G^{∗}C^{∗}T^{∗}G^{∗}G^{∗}A^{∗}T^{∗}C^{∗}A^{∗}A^{∗}C^{∗}A^{∗}T^{∗}+G^{∗}+G^{∗}+T (SEQIDNO.45), +A*+T*+G*T*G*A*C*T*G*A*G*T*C*C*+G*+C*+C (SEQIDNO.46), +G*+A*+A*C*T*C*T*G*T*G*A*G*C*T*C*C*+G (SEQIDNO.48), +C*+T*+G*G*T*G*G*A*C*T*A*A*C*A*+C*+A*+C (SEQIDNO.49), +T^{∗}+G^{∗}+G^{∗}A^{∗}C^{∗}C^{∗}T^{∗}G^{∗}A^{∗}C^{∗}A^{∗}A^{∗}G^{∗}G^{∗}+A^{∗}+G^{∗}+A (SEQIDNO.50), +C^{∗}+A^{∗}+C^{∗}A^{∗}C^{∗}G^{∗}G^{∗}C^{∗}A^{∗}T^{∗}G^{∗}T^{∗}A^{∗}+A^{∗}+G^{∗}+G (SEQIDNO.51), +G*+C*+C*A*G*A*T*A*G*G*C*T*A*A*+G*+T*+T (SEQIDNO.4), +A^{∗}+G^{∗}+G^{∗}C^{∗}T^{∗}A^{∗}G^{∗}T^{∗}C^{∗}T^{∗}C^{∗}G^{∗}A^{∗}C^{∗}+A^{∗}+G^{∗}+C (SEQIDNO.52), +G^{∗}+A^{∗}+T^{∗}C^{∗}C^{∗}T^{∗}C^{∗}A^{∗}T^{∗}G^{∗}A^{∗}T^{∗}A^{∗}G^{∗}+G^{∗}+C^{∗}+C (SEQIDNO.8), +G*+A*+T*C*G*C*A*G*T*G*C*C*G*C*+A*+A*+T (SEQIDNO.53), +G^{∗}A^{∗}T^{∗}C^{∗}G^{∗}C^{∗}A^{∗}G^{∗}T^{∗}G^{∗}C^{∗}C^{∗}G^{∗}C^{∗}+A^{∗}+A^{∗}+T (SEQIDNO.53), +C*+G*+T*G*T*T*G*T*A*A*C*C*T*C*+T*+T*+G (SEQIDNO.54), +G*+A*+T*C*G*C*A*G*T*G*C*C*+G*+C*+A (SEQIDNO.55), +G*+A*+T*C*G*C*A*G*T*G*C*C*G*+C*+A (SEQIDNO.55), +G*+G*+A*T*C*G*C*A*G*T*G*C*+C*+G*+C (SEQIDNO.56), +C*+T*+G*G*A*A*A*G*A*A*T*C*G*G*+A*+T*+C (SEQIDNO.5), wherein + are LNA, * are phosphorothioate internucleotide linkages, and optionally C is methyl-Cytosine
19. The ANGPTL4 inhibitor of item 18, wherein the LNA is beta-D-oxy LNA
20. The ANGPTL4 inhibitor of any of the previous items, wherein the inhibitor is coupled to a homing moiety that target the liver.
21. The ANGPTL4 inhibitor of any of the previous items, wherein the homing moiety is a GalNac moiety
22. The ANGPTL4 inhibitor of any of the previous items, wherein the GalNac moiety is a triantennary GalNac moiety.
23. The ANGPTL4 inhibitor according to any of items 17-20, wherein the homing moiety is attached to the inhibitor by a linker.
24. The ANGPTL4 inhibitor according to the previous item, wherein the linker is one of a nucleotide linker or an amino linker.
25. The ANGPTL4 inhibitor according to the previous item, wherein the linker is a nucleotide linker with a length of between 1 and 20 nucleotides
26. The ANGPTL4 inhibitor according to item 21 or 22, wherein the linker is an amino linker with a length of between NH-(CH2)2-12
27. The ANGPTL4 inhibitor according to the previous item, wherein the linker is a NH-(CH2)6 or a NH-(CH2)8 or a NH-(CH2)10 linker.
28. The ANGPTL4 inhibitor according to any of items 20-27, wherein the linker and homing moiety has the structure 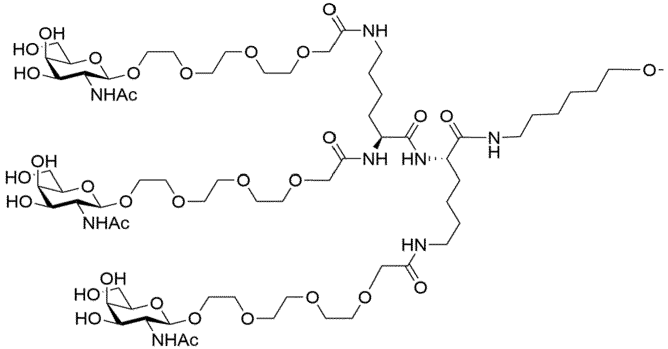
29. The ANGPTL4 inhibitor of any of the previous items, wherein the inhibitor is for use in combination with an ANGPTL3 inhibitor.
30. The ANGPTL4 inhibitor according to the previous item, wherein the ANGPTL3 inhibitor is an ANGPTL3 antibody and/or an ANGPTL3 antisense oligonucleotide
31. An ANGPTL4 inhibitor comprising the antisense oligonucleotide with the sequence +A*+G*+T*A*C*T*G*G*C*C*G*T*T*G*+A*+G*+G (SEQIDNO.186), wherein + are LNA such as beta-deoxy LNA, * are phosphorothioate internucleotide linkages, C is methyl-Cytocine, and wherein the inhibitor is attached to a homing moiety targeted to the liver.
32. The ANGPTL4 inhibitor of item 31, wherein the homing moiety is a GalNac molecule.
33. The ANGPTL4 inhibitor of item 31 or 32, wherein the GalNac molecule is a tri-antennary GalNac.
34. The ANGPTL4 inhibitor according to any of items 31-33, wherein the homing moiety is attached to the inhibitor by a linker.
35. The ANGPTL4 inhibitor according to item 34, wherein the linker is one of a nucleotide linker or an amino linker.
36. The ANGPTL4 inhibitor according to item 35, wherein the linker is a nucleotide linker with a length of between 1 and 20 nucleotides.
37. The ANGPTL4 inhibitor according to item 34 - 35, wherein the linker is an amino linker with the formula NH-(CH2)2-12.
38. The ANGPTL4 inhibitor according to the previous item, wherein the linker is a NH-(CH2)6, or NH-(CH2)8 or NH-(CH2)10 linker.
39. The ANGPTL4 inhibitor according to any of items 31-38, comprising the antisense oligonucleotide with the sequence +A*+G*+T*A*C*T*G*G*C*C*G*T*T*G*+A*+G*+G (SEQIDNO.186), wherein + are LNA such as beta-deoxy LNA, * are phosphorothioate internucleotide linkages, C is methyl-Cytocine, and wherein the inhibitor is attached to a triantennary GalNac moiety targeted to the liver, and a linker with formula NH-(CH2)6.
40. The ANGPTL4 inhibitor according to any of items 31-39, consisting of the antisense oligonucleotide with the sequence +A*+G*+T*A*C*T*G*G*C*C*G*T*T*G*+A*+G*+G (SEQIDNO.186), wherein + are LNA such as beta-deoxy LNA, * are phosphorothioate internucleotide linkages, C is methyl-Cytocine, and wherein the inhibitor is attached to a triantennary GalNac moiety targeted to the liver, and a linker with formula NH-(CH2)6.
41. The ANGPTL4 inhibitor according to any of items 31-40, wherein the antisense oligonucleotide consist of the sequence +A*+G*+T*A*C*T*G*G*C*C*G*T*T*G*+A*+G*+G (SEQIDNO.186), wherein + are LNA such as beta-deoxy LNA, * are phosphorothioate internucleotide linkages, C is methyl-Cytocine, and wherein the inhibitor is attached to a liver targeting homing moiety wherein linker and homing moiety has the structure 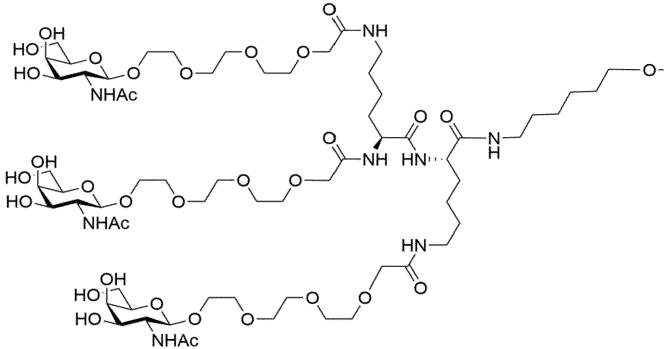
42. The ANGPTL4 inhibitor according to any of items 31 to 41, wherein the inhibitor is for use as a medicament
43. A pharmaceutical composition comprising the ANGPTL4 inhibitor according to any of items 31-41.
44. The ANGPTL4 inhibitor according to any of items 31 to 41, wherein the inhibitor is for use in a method of treatment.
45. The ANGPTL4 inhibitor according to any of items 31 to 44, wherein the inhibitor is for use in treatment, alleviation, pre-emptive treatment, prevention or prophylaxis of a cardiovascular disease in a mammal.
46. The ANGPTL4 inhibitor according to item 45, wherein the mammal is a human.
47. The ANGPTL4 inhibitor according to item 45 or 46, wherein the cardiovascular disease is atherosclerosis.
48. The ANGPTL4 inhibitor according to any of items 31-47, wherein the use is for lowering of plasma triglycerides and/or total cholesterol, such as for treatment of high levels of plasma triglycerides and/or high levels of total cholesterol.
49. The ANGPTL4 inhibitor according to any of items 31-47, wherein the use is for reducing circulating triglyceride-rich lipoproteins, such as for treatment of high levels of circulating triglyceride-rich lipoproteins.
50. The ANGPTL4 inhibitor according to any of items 31-47, wherein the use is for increasing plasma HDL-C levels, such as for treatment of low plasma HDL-C levels.
51. The ANGPTL4 inhibitor according to any of items 31-47, wherein the use is for reducing VLDL-ApoB production, such as for treatment of high VLDL-ApoB levels.
52. The ANGPTL4 inhibitor according to any of items 31-47, wherein the use is for prevention of atherosclerotic lesions.
53. The ANGPTL4 inhibitor according to any of items 31-47, wherein the use is for reducing atherosclerotic lesion size.
54. The ANGPTL4 inhibitor according to any of items 31-47, wherein the use is for reducing the number of severe atherosclerotic lesions.
55. The ANGPTL4 inhibitor according to any of items 31-47, wherein the use is for improving the atherosclerotic lesion index in a patient.
56. The ANGPTL4 inhibitor according to any of items 31-47, wherein the inhibitor inhibits the expression of ANGPTL4 mRNA.
57. The ANGPTL4 inhibitor according to any of items 31-56, wherein the inhibitor is for use in combination with another drug
58. The ANGPTL4 inhibitor according to any of items 31-57, wherein the inhibitor is for use in combination with an ANGPTL3 inhibitor
59. The ANGPTL4 inhibitor according to item 58, wherein the ANGPTL3 inhibitor is an antisense oligonucleotide or an antibody against ANGPTL3.
60. The ANGPTL4 inhibitor according to any of items 1-59, wherein the inhibitor is for use in a method where the inhibitor is for repeated administration to a primate, such as a human, for a period of at least 4 weeks.
61. The ANGPTL4 inhibitor according to item 60, wherein a continuous effective dosage is maintained in the human for a period of at least 4 weeks, such as at least 5 weeks such as at least any one of 6, 7, 8, 9, 10 weeks, such as at least 20 weeks or at least 25 weeks.
62. The ANGPTL4 inhibitor according to item 60 or 61, wherein the effective dosage is maintained for as long as needed.
63. The ANGPTL4 inhibitor according to items 60 - 62, wherein the inhibitor is for lowering plasma Total Cholesterol, non-HDL-C or plasma Triglyceride levels.
64. The ANGPTL4 inhibitor according to item 63, wherein the inhibitor is for use in combination with an ANGPTL3 inhibitor.

## Claims

1. An ANGPTL4 inhibitor comprising an antisense oligonucleotide with the sequence +A*+G*+T*A*C*T*G*G*C*C*G*T*T*G*+A*+G*+G (SEQ ID NO.186), wherein + are LNA such as beta-deoxy LNA, * are phosphorothioate internucleotide linkages, C is methyl-Cytocine, and wherein said inhibitor is attached to a homing moiety by an amino linker, wherein said homing moiety is targeted to the liver.

2. The ANGPTL4 inhibitor of claim 1, wherein the homing moiety is a GalNac molecule, such as a tri-antennary GalNac.

3. The ANGPTL4 inhibitor according to any of claims 1-2, wherein the homing moiety is attached to the inhibitor by an amino linker with the formula NH-(CH2)2-12..

4. The ANGPTL4 inhibitor according to claim 3, wherein the linker is an amino linker selected from the group consisting of a NH-(CH2)6 linker, NH-(CH2)8 linker and a NH-(CH2)10 linker.

5. The ANGPTL4 inhibitor according to claim 3, wherein the linker is an amino linker with the formula NH-(CH2)6.

6. The ANGPTL4 inhibitor according to any of claims 1 - 5, comprising the antisense oligonucleotide with the sequence +A*+G*+T*A*C*T*G*G*C*C*G*T*T*G*+A*+G*+G (SEQ ID NO.186), wherein + are LNA such as beta-deoxy LNA, * are phosphorothioate internucleotide linkages, C is methyl-Cytocine, and wherein the inhibitor is attached to a triantennary GalNac moiety targeted to the liver, and a linker with formula NH-(CH2)6.

7. The ANGPTL4 inhibitor according to any of claims 1 - 6, consisting of the antisense oligonucleotide with the sequence +A*+G*+T*A*C*T*G*G*C*C*G*T*T*G*+A*+G*+G (SEQ ID NO.186), wherein + are LNA such as beta-deoxy LNA, * are phosphorothioate internucleotide linkages, C is methyl-Cytocine, and wherein the inhibitor is attached to a triantennary GalNac moiety targeted to the liver, and a linker with formula NH-(CH2)6.

8. The ANGPTL4 inhibitor according to any of claims 1 - 7, wherein the antisense oligonucleotide consist of the sequence +A*+G*+T*A*C*T*G*G*C*C*G*T*T*G*+A*+G*+G (SEQ ID NO.186), wherein + are LNA such as beta-deoxy LNA, * are phosphorothioate internucleotide linkages, C is methyl-Cytocine, and wherein the inhibitor is attached to a liver targeting homing moiety wherein linker and homing moiety has the structure

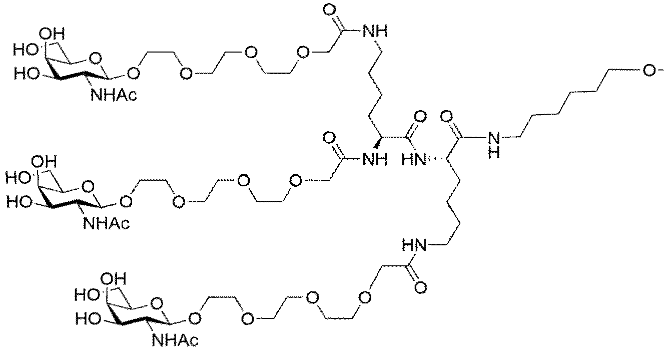

9. The ANGPTL4 inhibitor according to any of claims 1-8, wherein the inhibitor is for use as a medicament.

10. The ANGPTL4 inhibitor according to any of claims 1 - 9, wherein the inhibitor is for use in treatment, alleviation, pre-emptive treatment, prevention or prophylaxis of a cardiovascular disease in a mammal, such as atherosclerosis.

11. The ANGPTL4 inhibitor according to any of claims 1 - 10, wherein the use is for lowering of plasma triglycerides and/or total cholesterol and/or VLDL-ApoB production, such as for treatment of high levels of plasma triglycerides and/or high levels of total cholesterol and/or high levels of VLDL-ApoB.

12. The ANGPTL4 inhibitor according to any of claims 1 - 11, wherein the use is for increasing plasma HDL-C levels, such as for treatment of low plasma HDL-C levels.

13. The ANGPTL4 inhibitor according to any of claims 1 - 12, wherein the inhibitor inhibits the expression of ANGPTL4 mRNA.

14. The ANGPTL4 inhibitor according to any of claims 1 - 13, wherein the inhibitor is for use in combination with an ANGPTL3 inhibitor, such as an antisense oligonucleotide or an antibody against ANGPTL3.

15. The ANGPTL4 inhibitor according to claim 1 - 14, wherein a continuous effective dosage is maintained in a mammal such as a human for a period of at least 4 weeks, such as at least 5 weeks such as at least any one of 6, 7, 8, 9, 10 weeks, such as at least 20 weeks or at least 25 weeks.
